# EUROPEAN PATENT APPLICATION

(11) **EP 3 355 059 A2**
(43) Date of publication of application: **01.08.2018**
(21) Application number: 18162020.4
(22) Date of filing: 21.06.2010
(51) Int. Cl.: G01N 33/68

(54) **BIOMARKER ASSAY OF NEUROLOGICAL CONDITION**

(30) Priority: 19.06.2009 US 21872709 P; 17.05.2010 US 34518810 P
(62) Divisional of application: 10790319.7
(71) Applicant: Banyan Biomarkers, Inc., Alachua, FL 32615 (US)
(72) Inventor: SVETLOV, Stanislav I, Alachua, FL Florida 32615 (US); MARTINIS, Juan, Alachua, FL Florida 32615 (US); LARNER, Stephen Frank, Newberry, FL Florida 32669 (US); WANG, Kevin Ka-wang, Gainesville, FL Florida 32607 (US)
(74) Representative: ABG Patentes, S.L.

(57) **Abstract**

A process and assay for determining the neurological condition in a subject is provided whereby the level of one or more neuroactive biomarkers is measured in a sample obtained from the subject. The processes and assay include measurement of multiple neuroactive biomarkers for synergistic determination of a neurological condition such as neurological damage due to injury, disease, contact with a compound, or other source.

## Description

### GOVERNMENTAL SUPPORT

Portions of this work were supported by grants N14-06-1-1029, W81XWH-8-1-0376 and W81XWH-07-01-0701 from the United States Department of Defense.

### CROSS REFERENCE TO RELATED APPLICATIONS

This application claims priority to United States Provisional Application No. 61/218,727 filed June 19, 2009 and United States Provisional Application No. 61/345,188 filed May 17, 2010, the contents of each of which are incorporated herein by reference in their entirety.

### FIELD OF THE INVENTION

The present invention relates in general to determination of a neurological condition of an individual and in particular to measuring a quantity of a neuropredictive conditional biomarker(s) as a means to detect, diagnose, differentiate or treat a neurological condition.

### BACKGROUND OF THE INVENTION

The field of clinical neurology remains frustrated by the recognition that secondary injury to a central nervous system tissue associated with physiologic response to the initial insult could be lessened if only the initial insult could be rapidly diagnosed or in the case of a progressive disorder before stress on central nervous system tissues reached a preselected threshold. Traumatic, ischemic, and neurotoxic chemical insult, along with generic disorders, all present the prospect of brain damage. While the diagnosis of severe forms of each of these causes of brain damage is straightforward through clinical response testing and computed tomography (CT) and magnetic resonance imaging (MRI) testing, these diagnostics have their limitations in that spectroscopic imaging is both costly and time consuming while clinical response testing of incapacitated individuals is of limited value and often precludes a nuanced diagnosis. Additionally, owing to the limitations of existing diagnostics, situations under which a subject experiences a stress to their neurological condition such that the subject often is unaware that damage has occurred or seek treatment as the subtle symptoms often quickly resolve. The lack of treatment of these mild to moderate challenges to neurologic condition of a subject can have a cumulative effect or subsequently result in a severe brain damage event which in either case has a poor clinical prognosis.

In order to overcome the limitations associated with spectroscopic and clinical response diagnosis of neurological condition, there is increasing attention on the use of biomarkers as internal indicators of change as to molecular or cellular level health condition of a subject. As detection of biomarkers uses a sample obtained from a subject and detects the biomarkers in that sample, typically cerebrospinal fluid, blood, or plasma, biomarker detection holds the prospect of inexpensive, rapid, and objective measurement of neurological condition. The attainment of rapid and objective indicators of neurological condition allows one to determine severity of a non-normal brain condition on a scale with a degree of objectivity, predict outcome, guide therapy of the condition, as well as monitor subject responsiveness and recovery. Additionally, such information as obtained from numerous subjects allows one to gain a degree of insight into the mechanism of brain injury.

A number of biomarkers have been identified as being associated with severe traumatic brain injury as is often seen in vehicle collision and combat wounded subjects. These biomarkers have included spectrin breakdown products such as SBDP150, SBDP150i, SBDP145 (calpain mediated acute neural necrosis), SBDP120 (caspase mediated delayed neural apoptosis), UCH-L1 (neuronal cell body damage marker), and MAP-2 dendritic cell injury associated marker. The nature of these biomarkers is detailed in U.S. Patents 7,291,710 and 7,396,654, the contents of which are hereby incorporated by reference.

Glial Fibrillary Acidic Protein (GFAP), as a member of the cytoskeletal protein family, is the principal 8-9 nanometer intermediate filament glial cells such as in mature astrocytes of the central nervous system (CNS). GFAP is a monomeric molecule with a molecular mass between 40 and 53 kDa and an isoelectric point between 5.7 and 5.8. GFAP is highly brain specific protein that is not found outside the CNS. GFAP is released in response to brain injury and released into the blood and CSF soon after brain injury. In the CNS following injury, either as a result of trauma, disease, genetic disorders, or chemical insult, astrocytes become reactive in a way termed astrogliosis or gliosis that is characterized by rapid synthesis of GFAP. However, GFAP normally increases with age and there is a wide variation in the concentration and metabolic turnover of GFAP in brain tissue.

Thus, there exists a need for a process and an assay for providing improved measurement of neurological condition.

### SUMMARY OF THE INVENTION

A process is provided for detecting or distinguishing the severity of traumatic brain injury of a subject including measuring in a sample obtained at a first time from the subject a quantity of a first biomarker, illustratively GFAP, whereby said measuring determines the magnitude of traumatic brain injury of the subject. Increased levels of GFAP are indicative of TBI. In the absence of symptoms of severe-TBI, elevated levels of GFAP within 2 hours of injury are indicative of mild- or moderate-TBI. The quantity of a first biomarker is optionally correlated with CT scan normality, or GCS score. The inventive process allows distinguishing or detection of mild-TBI, moderate-TBI, severe-TBI, or the absence of TBI. Optionally, a quantity of one or more additional biomarkers is measured in the sample or in a second sample. An additional biomarker is optionally UCH-L1, NSE, MAP-2, SBDP150, SBDP145, SBDP120, or a control. A compound is optionally administered to a subject prior to obtaining a sample. A compound is illustratively kainic acid, MPTP, an amphetamine, cisplatin, or antagonists of a NMDA receptor. Measuring the quantity of one or more neuroactive biomarkers is optionally performed prior to 24 hours following injury alone or also after 24 hours following injury.

A process is provided for determining the neurological condition of a subject including measuring in a sample obtained at a first time from the subject a quantity of a first neuroactive biomarker whereby the measuring determines the neurological condition of the subject. A sample is optionally cerebrospinal fluid, blood, or a fraction thereof. The first neuroactive biomarker is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1.

In some embodiments an inventive process includes measuring a quantity of a second neuroactive biomarker. The second neuroactive biomarker is optionally measured at the same time as said first neuroactive biomarker. A first neuroactive biomarker is optionally UCH-L1 and a second neuroactive biomarker is GFAP, SBDP150, SBDP150i, SBDP145, SBDP120, NSE, S100β, MAP-2, MAP-1, MAP-3, MAP-4, MAP-5, MBP, Tau, NF-L, NF-M, NF-H, α-internexin, CB-1, CB-2; ICAM, VAM, NCAM, NL-CAM, AL-CAM, C-CAM; synaptotagmin, synaptophysin, synapsin, SNAP; CRMP-2, CRMP-1, CRMP-3, CRMP-4, iNOS, or βIII-tubulin. In some embodiments a first neuroactive biomarker is LC3 and a second neuroactive biomarker is MAP1. The quantity first neurological biomarker or the second neurological biomarker are optionally compared to the quantity of the biomarker in one or more other individuals with no known neurological damage. The first neurological biomarker and the second neurological biomarker are optionally in the same sample.

An assay for determining the neurological condition of a subject is provided including a substrate for holding a sample isolated from the subject and a first neuroactive biomarker specifically binding agent whereby reacting the first neuroactive biomarker specific binding agent with a portion of the biological sample is evidence of the neurological condition of the subject. A first neuroactive biomarker specific binding agent is optionally an antibody. An antibody optionally recognizes a neuroactive biomarker that is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1.

A process is provided for detecting a neurological condition in a subject following administration of a compound including administering a compound to a subject, obtaining a sample from said subject, and assaying said sample for the presence of a neuroactive biomarker that is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1, whereby said assaying allows detecting neurological damage in said subject. The sample is optionally serum, cerebrospinal fluid, or neuronal tissue. Neuronal tissue is optionally obtained from the cortex or hippocampus of the subject. A compound is optionally kainic acid, MPTP, an amphetamine, cisplatin, or antagonists of a NMDA receptor.

### BRIEF DESCRIPTION OF THE DRAWINGS

FIG. 1 illustrates GFAP and other biomarkers in control and severe TBI human subjects from initially taken CSF samples;
FIG. 2 illustrates GFAP and other biomarkers in the control and severe TBI human subjects of FIG. 1 in serum samples;
FIG. 3 illustrates GFAP and other biomarkers human control and severe TBI human subjects summarizing the data of FIGs. 1 and 2;
FIG. 4 illustrates arterial blood pressure (MABP), intracranial pressure (ICP) and cerebral profusion pressure (CPP) for a single human subject of traumatic brain injury as a function of time;
FIG. 5 represents biomarkers in CSF and serum samples from the single human subject of traumatic brain injury of FIG. 4 as a function of time;
FIG. 6 represents biomarkers in CSF and serum samples from another individual human subject of traumatic brain injury as a function of time;
FIG. 7 represents GFAP concentration for controls and individuals in a mild/moderate traumatic brain injury cohort as determined by CT scan in samples taken upon admission and 24 hours thereafter;
FIG. 8 represents parallel assays for UCH-L1 from the samples used for FIG. 7;
FIG. 9 illustrates the concentration of UCH-L1 and GFAP as well as S100β, provided as a function of injury magnitude between control, mild, and moderate traumatic brain injury;
FIG. 10 illustrates the concentration of the same markers as depicted in FIG. 9 with respect to initial evidence upon hospital admission as to lesions in tomography scans;
FIG. 11 represents UCH-L1, GFAP, S100β, NSE, MBP, and MAP2 amounts present in serum post severe traumatic brain injury in human subjects as a function of CT scan results;
FIG. 12 illustrates the levels of UCH-L1 by western blotting and ELISA in rat CSF or serum following CCI induced traumatic brain injury;
FIG. 13 illustrates relative GFAP expression in rat cortex (A) and hippocampus (B) following experimental blast-induced non-penetrating injury;
FIG. 14 illustrates relative CNPase expression in rat cortex (A) and hippocampus (B) following experimental blast-induced non-penetrating injury;
FIG. 15 illustrates GFAP levels in rat CSF (A) and serum (B) as measured by ELISA following experimental blast-induced non-penetrating injury;
FIG. 16 illustrates NSE levels in rat CSF (A) and serum (B) as measured by ELISA following experimental blast-induced non-penetrating injury;
FIG. 17 illustrates UCH-L1 levels in rat CSF (A) and plasma (B) as measured by ELISA following experimental blast-induced non-penetrating injury;
FIG. 18 illustrates CNPase levels in rat CSF as measured by western blot following experimental blast-induced non-penetrating injury;
FIG. 19 illustrates sICAM-1 levels in rat CSF (A) and serum (B) following experimental blast-induced non-penetrating injury;
FIG. 20 illustrates iNOS levels in rat plasma following experimental blast-induced non-penetrating injury;
FIG. 21 illustrates distribution of NeuN in rat (A) and human (B) tissues;
FIG. 22 illustrates NeuN and SBDP 150/145 in rat CSF following experimental blast-induced non-penetrating injury;
FIG. 23 illustrates NeuN in human CSF following traumatic brain injury;
FIG. 24 illustrates L-selectin in rat serum following experimental blast-induced non-penetrating injury;
FIG. 25 illustrates sICAM-1 levels in rat serum and CSF following experimental blast-induced non-penetrating injuries;
FIG. 26 illustrates β-NGF levels in rat serum following experimental blast-induced non-penetrating injuries;
FIG. 27 illustrates Neuropilin-2 levels in rat serum following experimental blast-induced non-penetrating injuries;
FIG. 28 illustrates Resistin levels in rat serum following experimental blast-induced non-penetrating injuries;
FIG. 29 illustrates Orexin levels in rat serum following experimental blast-induced non-penetrating injuries;
FIG. 30 illustrates Fractalkine levels in rat serum following experimental blast-induced non-penetrating injuries;
FIG. 31 illustrates Neuropilin-2 levels in rat cerebellum following experimental blast-induced non-penetrating injuries;
FIG. 32 illustrates SBDP145 levels in CSF (A) and serum (B) following sham, mild MCAO challenge, and severe MCAO challenge;

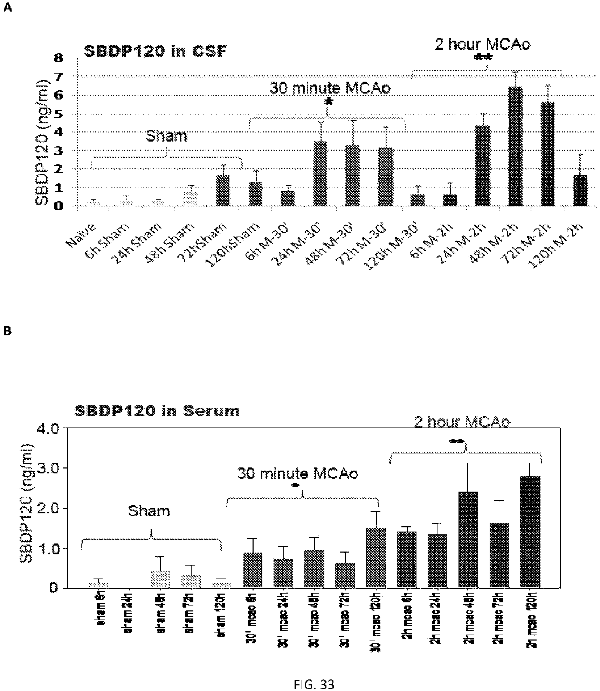

FIG. 33 illustrates SBDP120 levels in CSF (A) and serum (B) following sham, mild MCAO challenge, and severe MCAO challenge;
FIG. 34 represents MAP2 elevation in CSF (A) and serum (B) following sham, mild MCAO challenge, and severe MCAO challenge;
FIG. 35 represents UCH-L1 levels in serum following sham, mild MCAO challenge, and severe MCAO challenge;
FIG. 36 illustrates levels of SBDP145 (A), SBDP120 (B), and MAP-2 in plasma obtained from human patients suffering ischemic or hemorrhagic stroke;
FIG. 37 illustrates UCH-L1 levels in plasma obtained from human patients suffering ischemic or hemorrhagic stroke; and

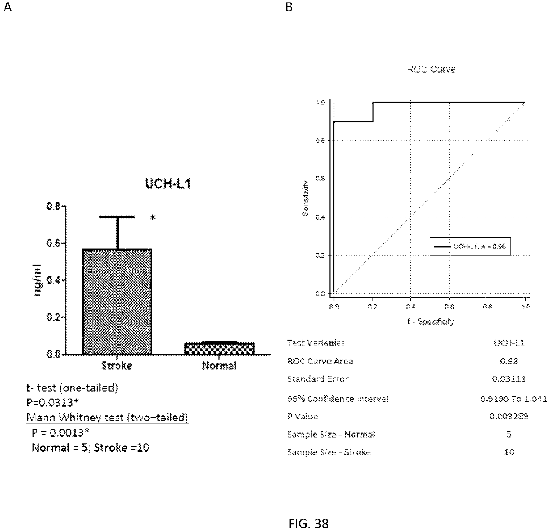

FIG. 38 illustrates the diagnostic utility of UCH-L1 for stroke.

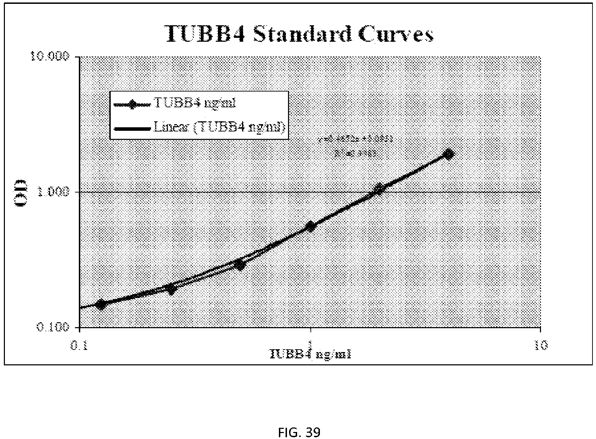

FIG. 39 illustrates a standard curve for an ELISA assay for TUBB4 as a biomarker.

### DESCRIPTION OF THE INVENTION

The present invention has utility in the diagnosis and management of abnormal neurological condition. Through the measurement of a neuroactive biomarker from a subject optionally in combination with values obtained for an additional neuroactive biomarker, a determination of subject neurological condition is provided with greater specificity than previously attainable.

The subject invention also has utility as a means of detecting neurological trauma or condition predictive or indicative of future disease or present or future injury. Illustratively, the invention has utility as a safety or efficacy screening protocol *in vivo* or *in vitro* for drug discovery or development. Drug discovery or development is not limited to drugs directed to neurological conditions. The neuroactive biomarkers optionally have utility to detect expected or unexpected neurological side effects in *in vivo* animal studies as a means of selecting a lead compound for analyses or as a means of assessing safety of a previously identified drug candidate.

A process for determining a neurological condition is provided that includes measuring the quantity of a first neuroactive biomarker in a sample. A neuroactive biomarker is a biomarker that is associated with, affected by, activated by, effects, or otherwise associates with a neuronal cell. The quantity of a neuroactive biomarker in a sample derived from a subject correlates with the presence or absence of a neurological condition.

The term "biomarker" as used herein represents antibodies, DNA, RNA, miRNA, fragments of RNA, fragments of DNA, peptides, proteins, lipids, or other biological material whose presence, absence, level or activity is correlative of or predictive of neurological condition, toxicity, damage, or disease.

A biomarker is optionally selective for detecting or diagnosing neurological conditions such as neurotoxic insult and others. Optionally, a biomarker is both specific and effective for the detection and distinguishing levels of chemical induced neurotoxicity. Such biomarkers are optionally termed neuroactive biomarkers.

A biomarker is illustratively a peptide or a protein. Detection of the presence or absence of protein, or increases or decreases in protein levels correlates with the presence or absence of a neurological condition such as neurological damage. As used herein, "peptide" means peptides of any length and includes proteins. The terms "polypeptide" and "oligopeptide" are used herein without any particular intended size limitation, unless a particular size is otherwise stated.

A biomarker is optionally a polynucleic acid such as an oligonucleotide. An oligonucleotide is a DNA or RNA molecule. Examples of RNA molecules illustratively include mRNA and miRNA molecules. RNA molecules were historically believed to have short half-lives in plasma. More recently, studies indicated that RNA molecules may be protected in plasma by protein or lipid vesicles. As such, RNA molecules released following or neurotoxic insult, for example, can be detected in cells, tissue, blood, plasma, serum, CSF, or other biological material and be associated with the presence of injury in the inventive method. Numerous methods are known in the art for isolating RNA from a biological sample. Illustratively, the methods described by El-Hefnaway, T, et al., Clinical Chem., 2004; 50(3);564-573, the contents of which are incorporated herein by reference, are operable in the present invention.

A biomarker is optionally a protein, optionally a full-length protein. Alternatively or in addition, an inventive biomarker is a portion of or the full length version of oligonucleotides or peptides that encode or are: GFAP, neuron specific enolase (NSE); ubiquitin C-terminal hydrolase L1 (UCHL1); Neuronal Nuclei protein (NeuN); 2', 3'-cyclic nucleotide 3'-phosphodiesterase (CNPase); Intercellular Adhesion Molecules (ICAMs), specifically ICAM-1, ICAM -2, and ICAM -5; Vascular Cell Adhesion Molecules (VCAM), specifically VCAM-1; neural Cell Adhesion Molecules (NCAM), specifically NCAM-1, NCAM-L1, NCAM-120, and NCAM-140; Neurolin-like cell adhesion molecule (NL-CAM); activated leukocyte cell adhesion molecule (AL-CAM); cell-cell adhesion molecules (C-CAM) (Frijns and Kappelle Stroke 2002: 33:2115), specifically C-CAM1; and inducible nitric oxide synthase (iNOS). An inventive neuroactive biomarker is optionally CNPase. A biomarker is illustratively any oligonucleotide encoding or a protein presented in Table 1, including fragments of a protein.

**Table 1**

| UCH-L1 | Glycogen phosphorylase, (BB-form)GP-BB | Precerebellin |
|---|---|---|
| MBP isoforms | CRMP-2 | Cortexin |
| SBDP150 (calpain) | NP25, NP22; Transgelin-3 | EMAP-II |
| SBDP120 (caspase) | SBDP150i (caspase) | Calcineurin-BDP |
| MBP-fragment (10/8K) | CaMPK-IIα | MAP2 |
| SBDP145 | MOG | N-Cadherin |
| Synaptophysin | PLP | N-CAM |
| βIII-Tubulin | PTPase (CD45) | Synaptobrevin |
| Tau-BDP-35K (calpain) | Nesprin-BDP | MAP1A (MAP1) |
| NF-L-BDP1 | OX-42 | MAP1B (MAP5) |
| NF-M-BDP1 | OX-8 | Prion-protein |
| NF-H-BDP1 | OX-6 | PEP19; PCP4 |
| Synaptotagmin | CaMPKIV | Synaptotagmin-BDP1 |
| PSD93-BDP1 | Dynamin | BDNF |
| AMPA-R-BDP1 | Clathrin HC | Nestin |
| NMDA-R-BDP | SNAP25 | IL-6 |
| SBDP150i (caspase) | Profilin (BDP?) | IL-10 |
| MAP2-BDP1 (calpain) | Cofilin (BDP?) | αII-spectrin SBDP 150+145 |
| MAP2-BDP2 (caspase) | APP-BDP (Calpain) | NG2; Phosphacan, neruocan; versican |
| alpha-synuclein | NSF | Ach Receptor fragment (Nicotinic, Muscarinic) |
| Synapsin 1 | IL-6 | I-CAM |
| Synapsin 2-BDP | MMP-9 | V-CAM |
| NeuN | S100β | AL-CAM |
| GFAP | Neuroglobin | CNPase |
| p24; VMP | UCH-L1 autoantibody | Neurofascins |
| PSD95 | Tau-BDP-35K (calpain) | Neuroserpin |
| α1,2-Tubulin | Tau-BDP-45K (caspase) | EAAT(1 and 2) |
| β1,2-Tubulin | Huntingtin-BDP-1 (calpain) | Nestin |
| Stathmin-2,3,4 (Dendritic) | Huntingtin-BDP-2 (caspase) | Synaptopodin |
| Striatin-BDP1 | Prion-protein BDP | |
| Snaptojanin-1,2-BDP1 | MBP (N-term half) | |
| betaIII-Spectrin | | β-synuclein |
| betaII-Spectrin-BDP110 (calpain) | Calbindin-9K | Resistin |
| betaII-Spectrin-BDP85 (caspase) | Tau-Total | Neuropilins |
| Cannabinoid-receptor1(CB1) | NSE | Orexin |
| Cannabinoid-receptor2(CB2) | CRMP-1 | Fracktalkine |
| MBP isoforms 14K+17K | CRMP-3 | β-NGF |
| Neurocalcin-delta (Glia) | CRMP-4 | L-selectin |
| Iba1 (Microglia) | CRMP-5 | iNOS |
| Peripherin (PNS) LC3 | Crerbellin 3 | DAT |

A biomarker is illustratively CNPase. CNPase is found in the myelin of the central nervous system. Neuron specific enolase (NSE) is found primarily in neurons. CNPase is a marker of oligodendrocyte lineage developing into Schwann cells producing myelin. CNPase is inventively observed in statistically significant increased levels following blast injury. The greatest levels of CNPase are observed between 1 hour and 30 days following blast injury, with greatest increases in the hippocampus. The levels of CNPase may increase over the first 30 days following injury suggesting an increase in Schwann cell development or myelin production. Following fluid percussion injury levels of CNPase colocalized with BrdU positive cells. Urrea, C. et al., Restorative Neurology and Neuroscience, 2007; 25:6576. CNPase is preferably used as a neuroactive biomarker of Schwann cell development from oligodendrocytes. Alterations in the levels of CNPase in particular neuronal tissues such as the hippocampus is indicative of neuronal changes that signal an effect of a screened drug candidate or as a safety or efficacy measure of chemical compound or other therapy effect.

CNPase is found in the myelin of the central nervous system. CNPase is optionally used as a marker for safety and efficacy screening for drug candidates. Illustratively, CNPase is operable as a marker of the protective, regenerative or disruption effects of test compounds. Optionally, drug screening is performed *in vitro.* CNPase levels are determined before, after, or during test compound or control administration to Schwann cells cultured alone or as a component of a co-culture system. Illustratively, Schwann cells are co-cultured with sensory neuronal cells, muscle cells, or glial cells such as astrocytes or oligodendrocyte precursor cells.

A biomarker is optionally a cell adhesion molecule (CAM). CAMs belong to the immunoglobulin gene family of cell-matrix or cell-cell interaction molecules. In the brain, they are particularly important in the cerebrovascular component of the blood brain barrier (BBB) and its interaction with the glia and neural cells (Frijns and Kappelle Stroke 2002: 33:2115). Cerebrovascular and BBB structure might be particularly at risk of traumatic and overpressure-induced brain injury or cerebral ischemia (e.g. stroke), leading to release of CAM into biofluids such as CSF or blood. Examples of CAM found in the brain might include soluble intercellular adhesion molecules (ICAM) e.g. ICAM-1, ICAM-2, ICAM-5, vascular cell adhesion molecules (VCAM) e.g. VCAM-1, Neural Cell Adhesion Molecules (NCAM), e.g. NCAM-1, NCAM-L1, NCAM-120, NCAM-140, Neurolin-like cell adhesion molecule (NL-CAM), and Activated Leukocyte cell adhesion molecule (AL-CAM) and cell-cell adhesion molecules(C-CAM), e.g. C-CAM1.

A biomarker is optionally NeuN or GFAP. NeuN is found in neuronal nuclei (Matevossian and Akbarian J Vis Exp. 2008; Oct 1;(20). pii:914). GFAP is a found primarily in astrocytic glial cells (numerous references, see Pekny M et al. Int Rev Neurobiol. 2007;82:95-111 for review). Lower levels of GFAP expression is also detected in non-myelinating Schwann cells and some mature Schwann cells undergoing 'de-differentiation' (Xu QG, Midha R, Martinez JA, Guo GF, Zochodne DW. Neuroscience. 2008 Apr 9;152(4):877-87).

Detection or quantification of one or more neuroactive biomarkers are illustratively operable to detect, diagnose, or treat a condition such as disease or injury, or screen for chemical or other therapeutics to treat a condition such as disease or injury. Diseases or conditions illustratively screenable include but are not limited to: myelin involving diseases such as multiple sclerosis, stroke, amyotrophic lateral sclerosis (ALS), chemotherapy, cancer, Parkinson's disease, nerve conduction abnormalities stemming from chemical or physiological abnormalities such as ulnar neuritis and carpel tunnel syndrome, other peripheral neuropathies illustratively including sciatic nerve crush (traumatic neuropathy), diabetic neuropathy, antimitotic-induced neuropathies (chemotherapy-induced neuropathy), experimental autoimmune encephalomyelitis (EAE), delayed-type hypersensitivity (DTH), rheumatoid arthritis, epilepsy, pain, neuropathic pain, traumatic neuronal injury such as traumatic brain injury, and intra-uterine trauma.

The detection of inventive biomarkers is also operable to screen potential drug candidates or analyze safety of previously identified drug candidates. These assays are optionally either *in vitro* or *in vivo. In vivo* screening or assay protocols illustratively include measurement of a neuroactive biomarker in an animal illustratively including a mouse, rat, or human. Studies to determine or monitor levels of neuroactive biomarker levels such as CNPase are optionally combined with behavioral analyses or motor deficit analyses such as: motor coordination tests illustratively including Rotarod, beam walk test, gait analysis, grid test, hanging test and string test; sedation tests illustratively including those detecting spontaneous locomotor activity in the open-field test; sensitivity tests for allodynia - cold bath tests, hot plate tests at 38°C and Von Frey tests; sensitivity tests for hyperalgesia - hot plate tests at 52°C and Randall-Sellito tests; and EMG evaluations such as sensory and motor nerve conduction, Compound Muscle Action Potential (CMAP) and h-wave reflex.

In some embodiments, an inventive process includes measuring the quantity of a first biomarker in a sample and measuring a quantity of a second biomarker. A second biomarker is optionally measured in the same sample as the first biomarker or a different sample. It is appreciated that the temporal nature of biomarker presence or activity is operable as an indicator or distinguisher of neurological condition. In a non-limiting example, the severity of experimental systemic exposure to MK-801, which causes Olney's lesions, correlates with the temporal maintenance of UCH-L1 in CSF. A second neuroactive biomarker is optionally measured at the same time or at a different time from the measurement of a first neuroactive biomarker. A different time is illustratively before or after detection of a first neuroactive biomarker. A second sample is optionally obtained before, after, or at the same time as the first sample. A second sample is optionally obtained from the same or a different subject.

First and second neuroactive biomarkers illustratively detect different conditions or the health or status of a different cell type. As a non-limiting example, GFAP is associated with glial cells such as astrocytes. An additional biomarker is optionally associated with the health of a different type of cell associated with neural function. Optionally, the other cell type is an axon, neuron, or dendrite. Through the use of an inventive assay inclusive of biomarkers associated with glial cells, and optionally with one other type of neural cell, the type of neural cells being stressed or killed as well as quantification of neurological condition results. Illustrative biomarkers associated with particular cell types or injury types are illustrated in Table 2.

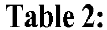

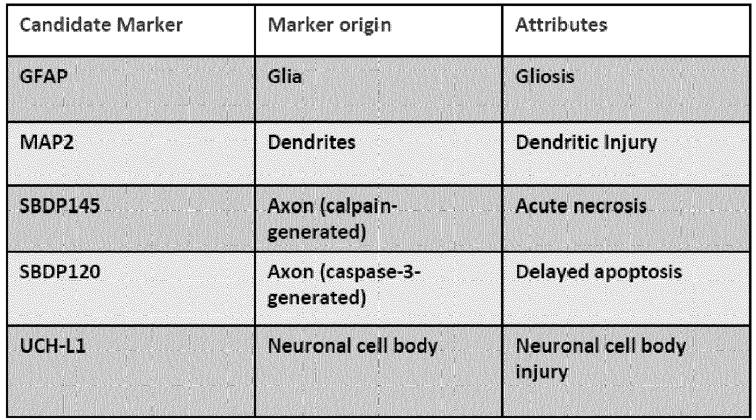

A synergistic measurement of a first neurological biomarker optionally along with at least one additional biomarker and comparing the quantity of the first neurological biomarker and the additional biomarker to each other or normal levels of the markers provides a determination of subject neurological condition. Specific biomarker levels that when measured in concert with a first neurological biomarker afford superior evaluation of subject neurological condition illustratively include SBDP145 (calpain mediated acute neural necrosis), SBDP120 (caspase mediated delayed neural apoptosis), UCH-L1 (neuronal cell body damage marker), and MAP-2 or other biomarker such as those listed in Table 1. Specific biomarker levels that when measured in concert with GFAP, for example, afford superior evaluation of subject neurological condition illustratively include SBDP145 and SBDP150 (calpain mediated acute neural necrosis), SBDP120 (caspase mediated delayed neural apoptosis), UCH-L1 (neuronal cell body damage marker), and MAP-2 (dendritic injury).

A first biomarker is optionally UCH-L1. Illustrative examples of second or additional biomarkers when UCH-L1 is a first biomarker illustratively include: GFAP; a SBDP illustratively including SBDP150, SBDP150i, SBDP145, and SBDP120; NSE, S100β; a MAP illustratively including MAP2, MAP1, MAP3, MAP4, and MAP5; MBP; Tau; Neurofilament protein (NF) such as NF-L, NF-M, NF-H and α-internexin; Canabionoid receptor (CB) such as CB-1, and CB-2; a cell adhesion molecule illustratively an ICAM, VAM, NCAM, NL-CAM, AL-CAM, and C-CAM; a synaptic protein illustratively Synaptotagmin, synaptophysin, synapsin, and SNAP; a CRMP illustratively CRMP-2, CRMP-1, CRMP-3 and CRMP-4; iNOS; βIII-tubulin or combinations thereof. Other first and second biomarkers illustratively include Nfasc186 and Nfasc155; LC3 and MAP1; or other combinations of any biomarker listed herein.

Biomarkers are optionally analyzed in combinations of multiple biomarkers in the same sample, samples taken from the same subject at the same or different times, or in a sample from a subject and another sample from another subject or a control subject. In addition to other combinations of biomarkers listed herein or recognized in the art, combinations illustratively include UCH-L1, GFAP, MAP-2, SBDP120, and SBDP145. In some embodiments a plurality of biomarkers are measured in the same sample, optionally simultaneously. In some embodiments a plurality of biomarkers are measured in separate samples. It is appreciated that some biomarkers are optionally measured in the same sample while other biomarkers are measured in other samples. Illustratively, some biomarkers are optionally measured in serum while the same or other biomarkers are measured in CSF, tissue, or other biological sample.

In some embodiments a plurality of biomarkers are analyzed to determine whether a neurological condition such as an ischemia or some level or severity of traumatic brain injury. Illustratively, to determine the severity of traumatic brain injury a plurality of biomarkers is UCH-L1, GFAP, MAP-2, SBDP120, and SBDP145. Illustratively, determining whether a stroke is ischemic a plurality of biomarkers is UCH-L1, GFAP, MAP-2, SBDP120, and SBDP145.

Analyses of an experimental blast injury to a subject revealed several inventive correlations between protein levels and the neurological condition resulting from neuronal injury. Neuronal injury is optionally the result of whole body blast, blast force to a particular portion of the body illustratively the head, or the result of other neuronal trauma or disease that produces detectable or differentiatable levels of neuroactive biomarkers. A number of experimental animal models have been implemented to study mechanisms of blast wave impact and include rodents and larger animals such as sheep. However, because of the rather generic nature of blast generators used in the different studies, the data on brain injury mechanisms and putative biomarkers have been difficult to analyze and compare until now.

To provide correlations between neurological condition and measured quantities of one or more neuroactive biomarkers, samples of CSF or serum, as two examples are collected from subjects with the samples being subjected to measurement of one or more neuroactive biomarkers. The subjects vary in neurological condition. Detected levels of one or more neuroactive biomarkers are then optionally correlated with CT scan results as well as GCS scoring. Based on these results, an inventive assay is developed and validated (Lee et al., Pharmacological Research 23:312-328, 2006, incorporated herein by reference).

Biomarker analyses are optionally performed using biological samples or fluids. Biological samples operable herein illustratively include, cells, tissues, cerebral spinal fluid (CSF), artificial CSF, whole blood, serum, plasma, cytosolic fluid, urine, feces, stomach fluids, digestive fluids, saliva, nasal or other airway fluid, vaginal fluids, semen, buffered saline, saline, water, or other biological fluid recognized in the art.

It is appreciated that neuroactive biomarkers, in addition to being obtained from CSF and serum, are also illustratively readily obtained from whole blood, plasma, saliva, urine, as well as solid tissue biopsy. While CSF is a preferred sampling fluid owing to direct contact with the nervous system, it is appreciated that other biological fluids have advantages in being sampled for other purposes and therefore allow for inventive determination of neurological condition as part of a battery of tests performed on a single sample such as blood, plasma, serum, saliva or urine.

After insult, nerve cells in *in vitro* culture or *in situ* in a subject express altered levels or activities of one or more biomarker proteins or oligonucleotide molecules than do such cells not subjected to the insult. Thus, samples that contain nerve cells, e.g., a biopsy of a central nervous system or peripheral nervous system tissue are suitable biological samples for use in the invention. In addition to nerve cells, however, other cells express illustratively all-spectrin including, for example, erythrocytes, cardiomyocytes, myocytes in skeletal muscles, hepatocytes, kidney cells and cells in testis. A biological sample including such cells or fluid secreted from these cells might also be used in an adaptation of the inventive methods to determine and/or characterize an injury to such non-nerve cells.

A biological sample is obtained from a subject by conventional techniques. For example, CSF is obtained by lumbar puncture. Blood is obtained by venipuncture, while plasma and serum are obtained by fractionating whole blood according to known methods. Surgical techniques for obtaining solid tissue samples are well known in the art. For example, methods for obtaining a nervous system tissue sample are described in standard neurosurgery texts such as Atlas of Neurosurgery: Basic Approaches to Cranial and Vascular Procedures, by F. Meyer, Churchill Livingstone, 1999; Stereotactic and Image Directed Surgery of Brain Tumors, 1st ed., by David G. T. Thomas, WB Saunders Co., 1993; and Cranial Microsurgery: Approaches and Techniques, by L. N. Sekhar and E. De Oliveira, 1st ed., Thieme Medical Publishing, 1999. Methods for obtaining and analyzing brain tissue are also described in Belay et al., Arch. Neurol. 58: 1673-1678 (2001); and Seijo et al., J. Clin. Microbiol. 38: 3892-3895 (2000).

Any subject that expresses an inventive biomarker is operable herein. Illustrative examples of a subject include a dog, a cat, a horse, a cow, a pig, a sheep, a goat, a chicken, non-human primate, a human, a rat, a mouse, and a cell. Subjects who benefit from the present invention are illustratively those suspected of having or at risk for developing abnormal neurological conditions, such as victims of brain injury caused by traumatic insults (e.g., gunshot wounds, automobile accidents, sports accidents, shaken baby syndrome), ischemic events (e.g., stroke, cerebral hemorrhage, cardiac arrest), neurodegenerative disorders (such as Alzheimer's, Huntington's, and Parkinson's diseases; prion-related disease; other forms of dementia), epilepsy, substance abuse (e.g., from amphetamines, Ecstasy/MDMA, or ethanol), and peripheral nervous system pathologies such as diabetic neuropathy, chemotherapy-induced neuropathy and neuropathic pain.

An exemplary process for detecting the presence or absence of one or more neuroactive biomarkers in a biological sample involves obtaining a biological sample from a subject, such as a human, contacting the biological sample with an agent capable of detecting of the marker being analyzed, illustratively including an antibody or aptamer, and analyzing binding of the agent optionally after washing. Those samples having specifically bound agent express the marker being analyzed.

An inventive process can be used to detect one or more neuroactive biomarkers in a biological sample *in vitro,* as well as *in vivo.* The quantity of expression of one or more other neuroactive biomarkers in a sample is compared with appropriate controls such as a first sample known to express detectable levels of the marker being analyzed (positive control) and a second sample known to not express detectable levels of the marker being analyzed (a negative control). For example, *in vitro* techniques for detection of a marker include enzyme linked immunosorbent assays (ELISAs), western blots, immunoprecipitation, and immunofluorescence. Also, *in vivo* techniques for detection of a marker illustratively include introducing a labeled agent that specifically binds the marker into a biological sample or test subject. For example, the agent can be labeled with a radioactive marker whose presence and location in a biological sample or test subject can be detected by standard imaging techniques.

Any suitable molecule that can specifically binds one or more neuroactive biomarkers are operative in the invention to achieve a synergistic assay. A neuroactive or other biomarker specifically binding agent is optionally an antibody capable of binding to the biomarker being analyzed. An antibody is optionally conjugated with a detectable label. Such antibodies can be polyclonal or monoclonal. An intact antibody, a fragment thereof (e.g., Fab or F(ab')₂), or an engineered variant thereof (e.g., sFv) can also be used. Such antibodies can be of any immunoglobulin class including IgG, IgM, IgE, IgA, IgD and any subclass thereof.

Antibody-based assays are illustratively used for analyzing a biological sample for the presence of one or more neuroactive biomarkers. Suitable western blotting methods are described herein or are known in the art. For more rapid analysis (as may be important in emergency medical situations), immunosorbent assays (e.g., ELISA and RIA) and immunoprecipitation assays may be used. As one example, the biological sample or a portion thereof is immobilized on a substrate, such as a membrane made of nitrocellulose or PVDF; or a rigid substrate made of polystyrene or other plastic polymer such as a microtiter plate, and the substrate is contacted with an antibody that specifically binds a neuroactive biomarker under conditions that allow binding of antibody to the biomarker being analyzed. After washing, the presence of the antibody on the substrate indicates that the sample contained the marker being assessed. If the antibody is directly conjugated with a detectable label, such as an enzyme, fluorophore, or radioisotope, the label presence is optionally detected by examining the substrate for the detectable label. A detectably labeled secondary antibody is optionally used that binds the marker-specific antibody is added to the substrate. The presence of detectable label on the substrate after washing indicates that the sample contained the marker.

Numerous permutations of these basic immunoassays are also operative in the invention. These include the biomarker-specific antibody, as opposed to the sample being immobilized on a substrate, and the substrate is contacted with a neuroactive biomarker conjugated with a detectable label under conditions that cause binding of antibody to the labeled marker. The substrate is then contacted with a sample under conditions that allow binding of the marker being analyzed to the antibody. A reduction in the amount of detectable label on the substrate after washing indicates that the sample contained the marker.

Although antibodies are illustrated herein for use in the invention because of their extensive characterization, any other suitable agent (e.g., a peptide, an aptamer, or a small organic molecule) that specifically binds a neuroactive biomarker is optionally used in place of the antibody. For example, an aptamer that specifically binds αII spectrin and/or one or more of its SBDPs might be used. Aptamers are nucleic acid-based molecules that bind specific ligands. Methods for making aptamers with a particular binding specificity are known as detailed in U.S. Patent Nos. 5,475,096; 5,670,637; 5,696,249; 5,270,163; 5,707,796; 5,595,877; 5,660,985; 5,567,588; 5,683,867; 5,637,459; and 6,011,020.

RNA and DNA binding antibodies are known in the art. Illustratively, an RNA binding antibody is synthesized from a series of antibody fragments from a phage display library. Illustrative examples of the methods used to synthesize RNA binding antibodies are found in Ye, J, et al., PNAS USA, 2008; 105:82-87 the contents of which are incorporated herein by reference as methods of generating RNA binding antibodies. As such, it is within the skill of the art to generate antibodies to RNA based biomarkers.

DNA binding antibodies are similarly well known in the art. Illustrative methods of generating DNA binding antibodies are found in Watts, RA, et al., Immunology, 1990; 69(3): 348-354 the contents of which are incorporated herein by reference as an exemplary method of generating anti-DNA antibodies.

A myriad of detectable labels are operative in a diagnostic assay for biomarker expression and are known in the art. Labels and labeling kits are commercially available optionally from Invitrogen Corp, Carlsbad, CA. Agents used in methods for detecting a neuroactive biomarker are optionally conjugated to a detectable label, e.g., an enzyme such as horseradish peroxidase. Agents labeled with horseradish peroxidase can be detected by adding an appropriate substrate that produces a color change in the presence of horseradish peroxidase. Several other detectable labels that may be used are known. Common examples include alkaline phosphatase, horseradish peroxidase, fluorescent molecules, luminescent molecules, colloidal gold, magnetic particles, biotin, radioisotopes, and other enzymes.

The present invention optionally includes a step of correlating the presence or amount of one or more other neuroactive biomarker in a biological sample with the severity and/or type of nerve cell injury. The amount of one or more neuroactive biomarkers in the biological sample is illustratively associated with neurological condition for traumatic brain injury. The results of an inventive assay to synergistically measure a first neuroactive biomarker and one or more additional neuroactive biomarkers help a physician determine the type and severity of injury with implications as to the types of cells that have been compromised. These results are in agreement with CT scan and GCS results, yet are quantitative, obtained more rapidly, and at far lower cost.

The present invention provides a step of comparing the quantity of one or more neuroactive biomarkers to normal levels to determine the neurological condition of the subject. It is appreciated that selection of one or more biomarkers allows one to identify the types of nerve cells implicated in an abnormal neurological condition as well as the nature of cell death illustratively a SBDP in the case of an axonal injury. The practice of an inventive process provides a test that can help a physician determine suitable therapeutics to administer for optimal benefit of the subject. While the subsequently provided data found in the examples is provided with respect to a full spectrum of traumatic brain injury, it is appreciated that these results are applicable to ischemic events, neurodegenerative disorders, prion related disease, epilepsy, chemical etiology and peripheral nervous system pathologies. A gender difference may be noted in an abnormal subject neurological condition.

An assay for analyzing cell damage in a subject is also provided. An exemplary process for detecting the presence or absence of one or more neuroactive biomarkers in a biological sample involves obtaining a biological sample from a subject, such as a human, contacting the biological sample with an agent capable of detecting of the biomarker being analyzed, illustratively including a primer, a probe, antigen, peptide, chemical agent, or antibody, and analyzing the sample for the presence of the biomarker. It is appreciated that other detection methods are similarly operable illustratively contact with a protein or nucleic acid specific stain.

An assay optionally includes: (a) a substrate for holding a sample isolated from a subject optionally suspected of having a damaged nerve cell, the sample or portion thereof being in fluid communication with the nervous system of the subject prior to being isolated from the subject; (b) a neuroactive biomarker specific binding agent; (c) a binding agent specific for another neurotactive biomarker; and (d) printed instructions for reacting: the neuroactive biomarker specific binding agent with the biological sample or a portion of the biological sample to detect the presence or amount of a neurological biomarker, and the agent specific for another neurotactive biomarker with the biological sample or a portion of the biological sample to detect the presence or amount of the at least one biomarker in the biological sample. The inventive assay can be used to detect neurological condition for financial renumeration.

The assay optionally includes a detectable label such as one conjugated to the agent, or one conjugated to a substance that specifically binds to the agent, such as a secondary antibody.

To provide correlations between a neurological condition and measured quantities of biomarkers, CSF or serum are optional biological fluids. Illustratively, samples of CSF or serum are collected from subjects with the samples being subjected to measurement of biomarkers. Collection of biological fluids or other biological samples are illustratively prior to or following administering a chemical or biological agent. Illustratively, a subject is optionally administered a chemical agent, such as an agent for drug screening. Prior to administration, at the time of administration, or any desired time thereafter, a biological sample is obtained from the subject. It is preferred that a biological sample is obtained during or shortly after the drug is found in the blood stream of the subject. Illustratively, a biological sample is obtained during the increase in plasma concentration observed following oral dosing. Illustratively, a biological sample is also obtained following peak plasma concentrations are obtained. Optionally, a biological sample is obtained 1, 2, 3, 4, 5, 10, 12, 24 hours or anytime in between after administration. Optionally, a biological sample is obtained 1, 2, 3, 4, 5, 6, 7, days or anytime in between. In some embodiments, a biological sample is obtained 1, 2, 3, 4, weeks or more, or any time in between. It is appreciated that neurotoxicity occurs immediately after administration or is delayed. A biological sample is optionally obtained 1, 2, 3, 6, months or more, or any time in between to detect delayed neurotoxicity. In some embodiments, a subject is continually dosed for hours, days, weeks, months, or years during which time one or more biological samples is obtained for biomarker screening. In some embodiments, phase IV trials are used to monitor the continued safety of a marketed chemical or biological agent. These trials optionally continue for years or indefinitely. As such, any time from prior to administration to years following the first administration, a biological sample is obtained for detection of one or more inventive biomarkers of neurotoxicity.

Baseline levels of biomarkers are those levels obtained in the target biological sample in the species of desired subject in the absence of a known neurological condition. These levels need not be expressed in hard concentrations, but may instead be known from parallel control experiments and expressed in terms of fluorescent units, density units, and the like. Typically, in the absence of a neurological condition, one or more SBDPs are present in biological samples at a negligible amount. However, UCH-L1 is a highly abundant protein in neurons. Determining the baseline levels of biomarkers illustratively including UCH-L1 or UCH-L1 biomarkers such as mRNA in neurons, plasma, or CSF, for example, of particular species is well within the skill of the art. Similarly, determining the concentration of baseline levels of other biomarkers is well within the skill of the art. Baseline levels are illustratively the quantity or activity of a biomarker in a sample from one or more subjects that are not suspected of having a neurological condition.

A biological sample is assayed by mechanisms known in the art for detecting or identifying the presence of one or more biomarkers present in the biological sample. Based on the amount or presence of a target biomarker in a biological sample, a ratio of one or more biomarkers is optionally calculated. The ratio is optionally the level of one or more biomarkers relative to the level of another biomarker in the same or a parallel sample, or the ratio of the quantity of the biomarker to a measured or previously established baseline level of the same biomarker in a subject known to be free of a pathological neurological condition. The ratio allows for the diagnosis of a neurological condition in the subject. An inventive process optionally administers a therapeutic to the subject that will either directly or indirectly alter the ratio of one or more biomarkers.

As used herein a "ratio" is either a positive ratio wherein the level of the target is greater than the target in a second sample or relative to a known or recognized baseline level of the same target. A negative ratio describes the level of the target as lower than the target in a second sample or relative to a known or recognized baseline level of the same target. A neutral ratio describes no observed change in target biomarker.

A neurological condition optionally results in or produces an injury. As used herein an "injury" is an alteration in cellular or molecular integrity, activity, level, robustness, state, or other alteration that is traceable to an event. Injury illustratively includes a physical, mechanical, chemical, biological, functional, infectious, or other modulator of cellular or molecular characteristics. An injury optionally results from an event. An event is illustratively, a physical trauma such as an impact (illustratively, percussive) or a biological abnormality such as a stroke resulting from either blockade (ischemic) or leakage (hemorrhagic) of a blood vessel. An event is optionally an infection by an infectious agent. A person of skill in the art recognizes numerous equivalent events that are encompassed by the terms injury or event.

An injury is optionally a physical event such as a percussive impact. An impact is optionally the like of a percussive injury such as resulting to a blow to the head, the body, or combinations thereof that either leave the cranial structure intact or results in breach thereof. Experimentally, several impact methods are used illustratively including controlled cortical impact (CCI) at a 1.6 mm depression depth, equivalent to severe TBI in human. This method is described in detail by Cox, CD, et al., J Neurotrauma, 2008; 25(11):1355-65, the contents of which are incorporated herein by reference. It is appreciated that other experimental methods producing impact trauma are similarly operable.

An may also result from stroke. Ischemic stroke is optionally modeled by middle cerebral artery occlusion (MCAO) in rodents. UCH-L1 protein levels, for example, are increased following mild MCAO which is further increased following severe MCAO challenge. Mild MCAO challenge may result in an increase of biomarker levels within two hours that is transient and returns to control levels within 24 hours. In contrast, severe MCAO challenge results in an increase in biomarker levels within two hours following injury and may be much more persistent demonstrating statistically significant levels out to 72 hours or more.

The invention employs a step of correlating the presence or amount of a biomarker in a biological sample with the severity and/or type of nerve cell (or other biomarker-expressing cell) toxicity. The amount of biomarker(s) in the biological sample directly relates to severity of neurological condition as a more severe injury damages a greater number of nerve cells which in turn causes a larger amount of biomarker(s) to accumulate in the biological sample (e.g., CSF; serum). Whether a neurotoxic insult triggers an apoptotic and/or necrotic type of cell death can also be determined by examining the biomarkers for SBDPs such as SBDP145 present in the biological sample. Necrotic cell death preferentially activates calpain, whereas apoptotic cell death preferentially activates caspase-3. Because calpain and caspase-3 SBDPs can be distinguished, measurement of these markers indicates the type of cell damage in the subject. For example, necrosis-induced calpain activation results in the production of SBDP150 and SBDP145; apoptosis-induced caspase-3 activation results in the production of SBDP150i and SBDP120; and activation of both pathways results in the production of all four markers. Also, the level of or kinetic extent of UCH-L1 biomarkers present in a biological sample may optionally distinguish mild injury from a more severe injury. In an illustrative example, severe MCAO (2h) produces increased UCH-L1 in both CSF and serum relative to mild challenge (30 min) while both produce UCH-L1 levels in excess of uninjured subjects. Moreover, the persistence or kinetic extent of the markers in a biological sample is indicative of the severity of the neurotoxicity with greater toxicity indicating increases persistence of UCH-L1 or SBDP biomarkers in the subject that is measured by an inventive process in biological samples taken at several time points following injury.

The results of such a test can help a physician determine whether the administration a particular therapeutic such as calpain and/or caspase inhibitors or muscarinic cholinergic receptor antagonists might be of benefit to a patient. This application may be especially important in detecting age and gender difference in cell death mechanism.

The invention optionally includes one or more therapeutic agents that may alter one or more characteristics of a target biomarker. A therapeutic optionally serves as an agonist or antagonist of a target biomarker or upstream effector of a biomarker. A therapeutic optionally affects a downstream function of a biomarker. For example, Acetylcholine (Ach) plays a role in pathological neuronal excitation and TBI-induced muscarinic cholinergic receptor activation may contribute to excitotoxic processes. As such, biomarkers optionally include levels or activity of Ach or muscarinic receptors. Optionally, an operable biomarker is a molecule, protein, nucleic acid or other that is effected by the activity of muscarinic receptor(s). As such, therapeutics operable in the subject invention illustratively include those that modulate various aspects of muscarinic cholinergic receptor activation.

Specific muscarinic receptors operable as therapeutic targets or modulators of therapeutic targets include the M₁, M₂, M₃, M₄, and M₅ muscarinic receptors.

The suitability of the muscarinic cholinergic receptor pathway in detecting and treating TBI arises from studies that demonstrated elevated ACh in brain cerebrospinal fluid (CSF) following experimental TBI (Gorman et al., 1989; Lyeth et al., 1993a) and ischemia (Kumagae and Matsui, 1991), as well as the injurious nature of high levels of muscarinic cholinergic receptor activation through application of cholinomimetics (Olney et al., 1983; Turski et al., 1983). Furthermore, acute administration of muscarinic antagonists improves behavioral recovery following experimental TBI (Lyeth et al., 1988a; Lyeth et al., 1988b; Lyeth and Hayes, 1992; Lyeth et al., 1993b; Robinson et al., 1990). As such chemical or biological agents that bind to, or alter a characteristic of a muscarinic cholinergic receptor are optionally screened for neurotoxicity of cells or tissues such as during target optimization in pre-clinical drug discovery.

A therapeutic compound, chemical compound, or biological compound, operable in the subject invention is illustratively any molecule, family, extract, solution, drug, pro-drug, or other that is operable for changing, optionally improving, therapeutic outcome of a subject at risk for or subjected to a neurotoxic insult. A therapeutic compound is optionally a muscarinic cholinergic receptor modulator such as an agonist or antagonist, an amphetamine. An agonist or antagonist may by direct or indirect. An indirect agonist or antagonist is optionally a molecule that breaks down or synthesizes acetylcholine or other muscarinic receptor related molecule illustratively, molecules currently used for the treatment of Alzheimer's disease. Cholinic mimetics or similar molecules are operable herein. An exemplary list of therapeutic compounds operable herein include: dicyclomine, scoplamine, milameline, N-methyl-4-piperidinylbenzilate NMP, pilocarpine, pirenzepine, acetylcholine, methacholine, carbachol, bethanechol, muscarine, oxotremorine M, oxotremorine, thapsigargin, calcium channel blockers or agonists, nicotine, xanomeline, BuTAC, clozapine, olanzapine, cevimeline, aceclidine, arecoline, tolterodine, rociverine, IQNP, indole alkaloids, himbacine, cyclostellettamines, derivatives thereof, pro-drugs thereof, and combinations thereof. A therapeutic compound is optionally a molecule operable to alter the level of or activity of a calpain or caspase. Such molecules and their administration are known in the art. It is appreciated that a compound is any molecule including molecules of less than 700 Daltons, peptides, proteins, nucleic acids, or other organic or inorganic molecules that is contacted with a subject, or portion thereof.

A compound is optionally any molecule, protein, nucleic acid, or other that alters the level of a neuroactive biomarker in a subject. A compound is optionally an experimental drug being examined in pre-clinical or clinical trials, or is a compound whose characteristics or affects are to be elucidated. A compound is optionally kainic acid, MPTP, an amphetamine, cisplatin or other chemotherapeutic compounds, antagonists of a NMDA receptor, any other compound listed herein, pro-drugs thereof, racemates thereof, isomers thereof, or combinations thereof. Example amphetamines include: ephedrine; amphetamine aspartate monohydrate; amphetamine sulfate; a dextroamphetamine, including dextroamphetamine saccharide, dextroamphetamine sulfate; methamphetamines; methylphenidate; levoamphetamine; racemates thereof; isomers thereof; derivatives thereof; or combinations thereof. Illustrative examples of antagonists of a NMDA receptor include those listed in Table 3 racemates thereof, isomers thereof, derivatives thereof, or combinations thereof:

**Table 3:**

| | | |
|---|---|---|
| AP-7 (drug) | Gacyclidine | PEAQX |
| AP5 | Hodgkinsine | Perzinfotel |
| Amantadine | Huperzine A | Phencyclidine |
| Aptiganel | Ibogaine | 8A-PDHQ |
| CGP-37849 | Ifenprodil | Psychotridine |
| DCKA | Indantadol | Remacemide |
| Delucemine | Ketamine | Rhynchophylline |
| Dexanabinol | Kynurenic acid | Riluzole |
| Dextromethorphan | Lubeluzole | Sabeluzole |
| Dextrorphan | Memantine | Selfotel |
| Dizocilpine | Midafotel | Tiletamine |
| Eliprodil | Neramexane | Xenon |
| Esketamine | Nitrous oxide | |
| Ethanol | | |
| NEFA | | |

As used herein the term "administering" is delivery of a compound to a subject. The compound is a chemical or biological agent administered with the intent to ameliorate one or more symptoms of a condition or treat a condition. A therapeutic compound is administered by a route determined to be appropriate for a particular subject by one skilled in the art. For example, the therapeutic compound is administered orally, parenterally (for example, intravenously, by intramuscular injection, by intraperitoneal injection, intratumorally, by inhalation, or transdermally. The exact amount of therapeutic compound required will vary from subject to subject, depending on the age, weight and general condition of the subject, the severity of the neurological condition that is being treated, the particular therapeutic compound used, its mode of administration, and the like. An appropriate amount may be determined by one of ordinary skill in the art using only routine experimentation given the teachings herein or by knowledge in the art without undue experimentation.

Processes of detecting or distinguishing the magnitude of traumatic brain injury (TBI) is also provided. Traumatic brain injury is illustratively mild-TBI, moderate-TBI, or severe-TBI. As used herein mild-TBI is defined as individuals presenting with a CGS score of 12-15 or any characteristic described in the National Center for Injury Prevention and Control, *Report to Congress on Mild Traumatic Brain Injury in the United States: Steps to Prevent a Serious Public Health Problem.* Atlanta, GA: Centers for Disease Control and Prevention; 2003, incorporated herein by reference. Moderate-TBI is defined as presenting a GCS score of 9-11. Severe-TBI is defined as presenting a GCS score of less than 9, presenting with an abnormal CT scan or by symptoms including unconsciousness for more than 30 minutes, post traumatic amnesia lasting more than 24 hours, and penetrating cranialcerebral injury.

A process of detecting or distinguishing between mild- or moderate-TBI illustratively includes obtaining a sample from a subject at a first time and measuring a quantity of GFAP in the sample where an elevated GFAP level indicates the presence of traumatic brain injury. The inventive process is optionally furthered by correlating the quantity of GFAP with CT scan normality or GCS score. A positive correlation for mild-TBI is observed when the GCS score is 12 or greater, and GFAP levels are elevated. Alternatively or in addition, a positive correlation for mild-TBI is observed when the CT scan results are abnormal, and GFAP levels are elevated. A positive correlation for moderate-TBI is observed when the GCS score is 9-11 and GFAP levels are elevated. Alternatively or in addition, a positive correlation for moderate-TBI is observed when the CT scan results are abnormal, and GFAP levels are elevated. Abnormal CT scan results are illustratively the presence of lesions. Unremarkable or normal CT scan results are the absence of lesions.

The levels of GFAP are optionally measured in samples obtained within 24 hours of injury. Optionally, GFAP levels are measured in samples obtained 0-24 hours of injury inclusive of all time points therebetween. In some embodiments a second sample is obtained at or beyond 24 hours following injury and the quantity of GFAP alone or along with an additional biomarker are measured.

A process for detecting or distinguishing between mild- or moderate-TBI optionally includes measuring a quantity of a second neuroactive biomarker. A second neuroactive biomarker is optionally any biomarker listed in Table 1. Optionally, a second neuroactive biomarker is UCH-L1, NSE, MAP2, SBDP150, SBDP150i, SBDP145, SBDP120, or a control biomarker illustratively S100β. Illustratively, the levels of UCH-L1 are elevated at one time point and reduced at a later time point following injury. Illustratively, one or more samples are obtained from a subject within two hours following injury, although other times prior to 24 hours are similarly operable. The biological sample(s) is assayed and the quantity of GFAP alone or along with UCH-L1 are measured. Elevated GFAP and UCH-L1 at a time less than 24 hours following injury along with reduced levels at or beyond 24 hours after injury is indicative of mild- or moderate-TBI. Sustained levels of one or more neuroactive biomarkers longer than 24 hours is indicative of severe-TBI.

A compound is illustratively administered to a subject either as a potential therapeutic or as a compound with known or unknown neurotoxic effect. A compound is illustratively any compound listed herein optionally kainic acid, MPTP, an amphetamine, cisplatin or other chemotherapeutics, antagonists of a NMDA receptor, combinations thereof, derivatives thereof, racemates thereof, or isomers thereof. Optionally, administration of a compound is an injury.

The practice of an inventive processes provides a test that can help a physician determine suitable therapeutic compound(s) to administer for optimal benefit of the subject. While the subsequently provided data found in the examples is provided with respect to a full spectrum of brain injury, it is appreciated that these results are applicable to ischemic events, neurodegenerative disorders, prion related disease, epilepsy, chemical or biological agent etiology, and peripheral nervous system pathologies. A gender difference may be present in abnormal subject neurological condition.

### Additional aspects of the invention

1. A process for determining the severity of traumatic brain injury of a subject comprising:
   measuring a quantity of GFAP in a sample obtained at a first time from the subject whereby said measuring determines the severity of traumatic brain injury of the subject.
2. The process of aspect 1 further comprising correlating said quantity of GFAP with CT scan normality, or GCS score.
3. The process of aspect 1 wherein said severity of brain injury is no traumatic brain injury, mild traumatic brain injury, moderate traumatic brain injury.
4. The process of aspect 1 further comprising measuring a quantity of one or more additional biomarkers.
5. The process of aspect 4 wherein said additional biomarker is UCH-L1, NSE, MAP-2, SBDP150, SBDP145, SBDP120, a control, or combinations thereof.
6. The process of aspect 1 wherein said level of traumatic brain injury is severe traumatic brain injury.
7. The process of aspect 1 further comprising administering a compound to said subject prior to said measuring.
8. The process of aspect 1 wherein said first time is 2 or fewer hours following injury.
9. A process for determining the neurological condition of a subject comprising:
   measuring a quantity of a first neuroactive biomarker in a sample obtained at a first time from the subject whereby said measuring determines the neurological condition of the subject.
10. The process of aspect 9 wherein said neurological condition is ischemia.
11. The process of aspect 8 wherein the first neuroactive biomarker is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1.
12. The process of aspect 9 further comprising measuring a quantity of a second neuroactive biomarker.
13. The process of aspect 12 wherein said first neuroactive biomarker is UCH-L1 and said second biomarker is GFAP, SBDP150, SBDP150i, SBDP145, SBDP120, NSE, S100β, MAP2, MAP1, MAP3, MAP4, MAP5, MBP, Tau, NF-L, NF-M, NF-H, α-internexin, CB-1, CB-2; ICAM, VAM, NCAM, NL-CAM, AL-CAM, C-CAM; synaptotagmin, synaptophysin, synapsin, SNAP; CRMP-2, CRMP-1, CRMP-3, cRMP-4 iNOS, βIII-tubulin, or a control.
14. The process of aspects 4 or 12 wherein said second neuroactive biomarker is measured at the same time as said first neuroactive biomarker.
15. The process of aspect 12 wherein said first neuroactive biomarker is LC3 and said second neuroactive biomarker is MAP1.
16. The process of aspect 12 wherein said first neuroactive biomarker is GFAP and the second neuroactive biomarker is UCH-L1, NSE, MAP2, SBDP150, SBDP145, or SBDP120.
17. The process of aspects 1, 9, or 12 further comprising comparing the quantity of said first neuroactive biomarker in said subject to other individuals with no known neurological damage.
18. The process of aspects 4 or 12 further comprising comparing the quantity of said second neuroactive biomarker in said subject to other individuals with no known neurological damage.
19. The process of aspects 4 or 12 wherein said first neurological biomarker and said second neurological biomarker are in the same sample.
20. An assay for determining the neurological condition of a subject comprising:
   a substrate for holding a biological sample isolated from the subject;
   a first neuroactive biomarker specifically binding agent;
   whereby reacting said first neuroactive biomarker specific binding agent with a portion of the biological sample is evidence of the neurological condition of the subject.
21. The assay of aspect 20 wherein the first neuroactive biomarker specific binding agent is an antibody.
22. The assay of aspect 21 wherein the antibody recognizes a neuroactive biomarker that is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1.
23. A process for detecting a neurological condition in a subject following administration of a compound comprising:
   administering a compound to a subject;
   obtaining a sample from said subject;
   assaying said sample for the presence of a neuroactive biomarker that is UCH-L1, GFAP, NSE, NeuN, CNPase, CAM-1, iNOS, MAP-1, MAP-2, SBDP145, SBDP120, βIII-tubulin, a synaptic protein, neuroserpin, α-internexin, LC3, neurofacin; an EAAT, DAT, nestin, cortin-1, CRMP, ICAM-1, ICAM-2, ICAM-5, VCAM-1, NCAM-1, NCAM-L1, NCAM-120, NCAM-140, NL-CAM, AL-CAM, or C-CAM1, whereby said assaying allows detecting neurological damage in said subject.
24. The process of aspects 7 or 23 wherein said compound is kainic acid, MPTP, an amphetamine, cisplatin, or antagonists of a NMDA receptor.
25. The process of aspect 24 wherein said amphetamine is methamphetamine.
26. The process of aspects 1, 9, 20, or 23 wherein said sample is blood or a fraction thereof, cerebrospinal fluid, or neuronal tissue.
27. The process of aspect 26 wherein said neuronal tissue is obtained from the cortex or hippocampus of said subject.

Various aspects of the present invention are illustrated by the following non-limiting examples. The examples are for illustrative purposes and are not a limitation on any practice of the present invention. It will be understood that variations and modifications can be made without departing from the spirit and scope of the invention. While the examples are generally directed to mammalian tissue, specifically, analyses of rat tissue, a person having ordinary skill in the art recognizes that similar techniques and other techniques know in the art readily translate the examples to other mammals such as humans. Reagents illustrated herein are commonly cross reactive between mammalian species or alternative reagents with similar properties are commercially available, and a person of ordinary skill in the art readily understands where such reagents may be obtained.

### Example 1

Materials for Biomarker Analyses. Sodium bicarbonate, (Sigma Cat #: C-3041), blocking buffer (Startingblock T20-TBS) (Pierce Cat#: 37543), Tris buffered saline with Tween 20 (TBST; Sigma Cat #: T-9039). Phosphate buffered saline (PBS; Sigma Cat #: P-3813); Tween 20 (Sigma Cat #: P5927); Ultra TMB ELISA (Pierce Cat #: 34028); and Nunc maxisorp ELISA plates (Fisher). Monoclonal and polyclonal UCH-L1 antibodies are made in-house or are obtained from Santa Cruz Biotechnology, Santa Cruz, CA. Antibodies directed to all-spectrin and breakdown products (SBDP) as well as to MAP2 are available from Santa Cruz Biotechnology, Santa Cruz, CA. Labels for antibodies of numerous subtypes are available from Invitrogen, Corp., Carlsbad, CA. Protein concentrations in biological samples are determined using bicinchoninic acid microprotein assays (Pierce Inc., Rockford, IL, USA) with albumin standards. All other necessary reagents and materials are known to those of skill in the art and are readily ascertainable.

Biomarker specific rabbit polyclonal antibodies and monoclonal antibodies are produced in the laboratory. To determine reactivity specificity of the antibodies a tissue panel is probed by western blot.

An indirect ELISA is used with the recombinant biomarker protein attached to the ELISA plate to determine optimal concentration of the antibodies used in the assay. This assay determines suitable concentrations of biomarker specific binding agent to use in the assay. Microplate wells are coated with rabbit polyclonal antihuman biomarker antibody. After determining concentration of rabbit antihuman biomarker antibody for a maximum signal, maximal detection limit of the indirect ELISA for each antibody is determined. An appropriate diluted sample is incubated with a rabbit polyclonal antihuman biomarker antibody (capture antibody) for 2 hours and then washed. Biotin labeled monoclonal antihuman biomarker antibody is then added and incubated with captured biomarker. After thorough wash, streptavidin horseradish peroxidase conjugate is added. After 1 hour incubation and the last washing step, the remaining conjugate is allowed to react with substrate of hydrogen peroxide tetramethyl benzadine. The reaction is stopped by addition of the acidic solution and absorbance of the resulting yellow reaction product is measured at 450 nanometers. The absorbance is proportional to the concentration of the biomarker. A standard curve is constructed by plotting absorbance values as a function of biomarker concentration using calibrator samples and concentrations of unknown samples are determined using the standard curve.

### Example 2

Severe Traumatic Brain Injury Study - 46 subjects suffering severe traumatic brain injury are studied for biomarker levels in various tissues and at various times following injury. Each of these subjects is over age 18, has a GCS of less than or equal to 8, and required ventriculostomy and neuromonitoring are performed as part of routine care. Control group A, synonymously detailed as CSF controls, includes 10 individuals also being over the age of 18 or older and no injuries. Samples are obtained during spinal anesthesia for routine surgical procedures, or access to CSF is associated with treatment of hydrocephalus or meningitis. A control group B, synonymously described as normal controls, totals 64 individuals, each age 18 or older and experiencing multiple injuries without brain injury. Further details with respect to the demographics of the study are provided in Table 4.

**Table 4. Subject Demographics for Severe Traumatic Brain Injury Study**

| | | **TBI** | **CSF Controls** | **Normal Controls** |
|---|---|---|---|---|
| Number | | 46 | 10 | 64 |
| | Males | 34 (73.9%) | 29 (65.9%) | 26 (40.6%) |
| | Females | 12 (26.1%) | 15 (34.1%) | 38 (59.4% |
| Age: | Average | 50.2 | 58.2 1,2 | 30.09 2,3 |
| | Std Dev | 19.54 | 20.52 | 15.42 |
| | Minimum | 19 | 23 | 18 |
| | Maximum | 88 | 82 | 74 |
| Race: | Caucasian | | | |
| | Black | 45 | 38 (86.4%) | 52 (81.2%) |
| | Asian | 1 | 6 (13.6) | 4 (6.3%) |
| | Other | | | 7 (10.9%) |
| | | | | 1 (1.6%) |

| GCS in Emergency Department | | | | |
|---|---|---|---|---|
| | Average | 5.3 | | |
| | Std Dev | 1.9 | | |

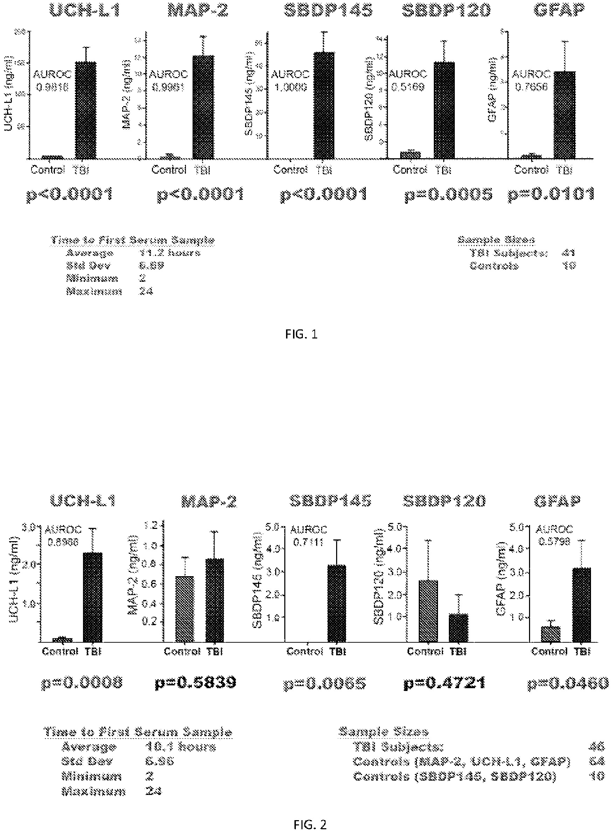

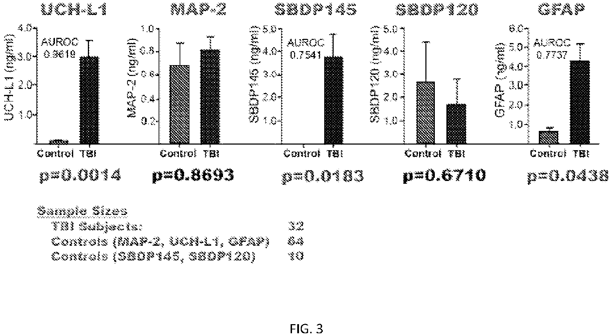

The levels of biomarkers found in the first available CSF and serum samples obtained in the study are analyzed by ELISA essentially as described in Example 1 with the recombinant biomarker replaced by sample and results are provided in FIGs. 1 and 2, respectively. The average first CSF sample collected as detailed in FIG. 1 is 11.2 hours while the average time for collection of a serum sample subsequent to injury event as per FIG. 2 is 10.1 hours. The quantity of each of biomarkers UCH-L1, MAP-2, SBDP145, SBDP120, and GFAP are provided for each sample for the cohort of traumatic brain injury sufferers as compared to a control group. The diagnostic utility of the various biomarkers within the first 12 hours subsequent to injury based on a compilation of CSF and serum data is provided in FIG. 3 and indicates in particular the value of GFAP as well as that of additional markers UCH-L1 and the spectrin breakdown products. Elevated levels of UCH-L1 are indicative of the compromise of neuronal cell body damage while an increase in SPDP145 with a corresponding decrease in SPDP120 is suggestive of acute axonal necrosis.

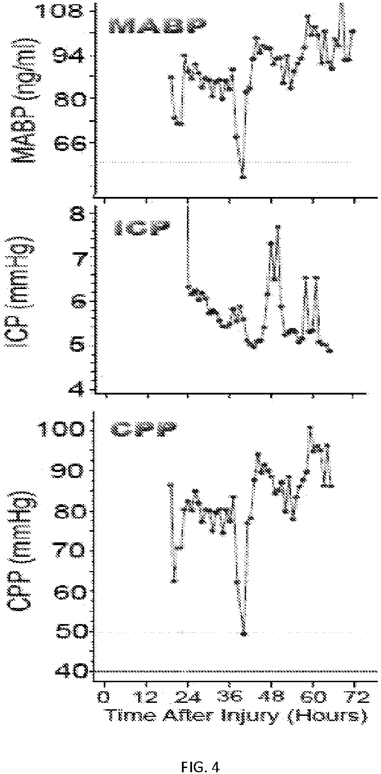

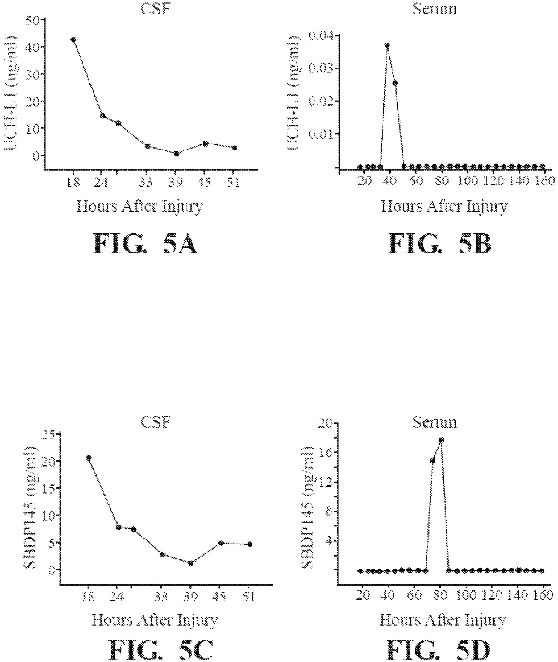

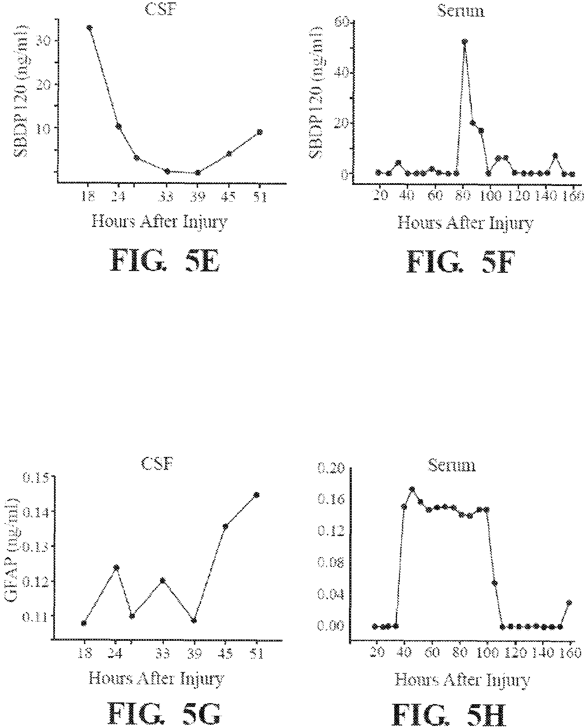

One subject from the traumatic brain injury cohort was a 52 year old Caucasian woman who had been involved in a motorcycle accident while not wearing a helmet. Upon admission to an emergency room her GCS was 3 and during the first 24 hours subsequent to trauma her best GCS was 8. After 10 days her GCS was 11. CT scanning revealed SAH and facial fractures with a Marshall score of 11 and a Rotterdam score of 2. Ventriculostomy was removed after 5 years and an overall good outcome was obtained. Arterial blood pressure (MABP), intracranial pressure (ICP) and cerebral profusion pressure (CPP) for this sufferer of traumatic brain injury as a function of time is depicted in FIG. 4. A possible secondary insult is noted at approximately 40 hours subsequent to the injury as noted by a drop in MABP and CPP. The changes in concentration of inventive biomarkers per CSF and serum samples from this individual are noted in FIG. 5. These results include a sharp increase in GFAP in both the CSF and serum as well as the changes in the other biomarkers depicted in FIG. 5 and provide important clinical information as to the nature of the injury and the types of cells involved, as well as modes of cell death associated with the spectrin breakdown products.

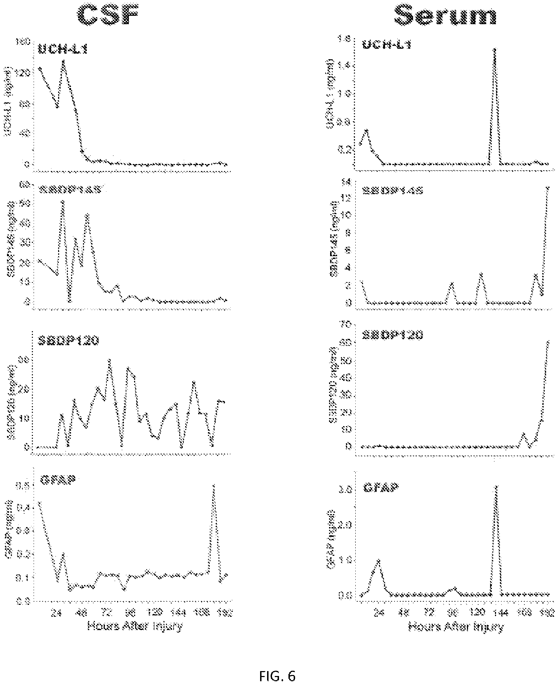

Another individual of the severe traumatic brain injury cohort included a 51 year old Caucasian woman who had suffered a crush injury associated with a horse falling on the individual. GCS on admission to emergency room was 3 with imaging analysis initially being unremarkable with minor cortical and subcortical contusions. MRI on day 5 revealed significant contusions in posterior fossa. The Marshall scale at that point was indicated to be 11 with a Rotterdam scale score of 3. The subject deteriorated and care was withdrawn 10 days after injury. The CSF and serum values for this individual during a period of time are provided in FIG. 6.

The concentration of spectrin breakdown products, MAP-2 and UCH-L1 as a function of time subsequent to traumatic brain injury has been reported elsewhere as exemplified in U.S. Patents 7,291,710 and 7,396,654 each of which is incorporated herein by reference.

An analysis was performed to evaluate the ability of biomarkers measured in serum to predict TBI outcome, specifically GCS. Stepwise regression analysis is used to evaluate biomarkers as an independent predictive factor, along with the demographic factors of age and gender, and also interactions between pairs of factors. Interactions determine important predictive potential between related factors, such as when the relationship between a biomarker and outcome may be different for men and women, such a relationship would be defined as a gender by biomarker interaction.

The resulting analysis identifies biomarkers UCH-L1, MAP-2, and GFAP as being statistically significant predictors of GCS (Tables 5, 6). Furthermore, GFAP has improved predictability when evaluated in combination with UCH-L1 and gender (Tables 7, 8).

**Table 5. Stepwise Regression Analysis 1 - Cohort includes:**

| All Subjects >= 18 Years Old | | | | |
|---|---|---|---|---|
| Summary of Stepwise Selection - 48 Subjects | | | | |
| Variable | Parameter | Model | | |
| Step Entered | Estimate | R-Square | F Value | p-value |
| Intercept | 13.02579 | | | |
| 2 SEXCD | -2.99242 | 0.1580 | 7.29 | 0.0098 |
| 1 CSF_UCH_L1 | -0.01164 | 0.2519 | 11.54 | 0.0015 |
| 3 Serum_MAP_2 | 0.96055 | 0.3226 | 4.59 | 0.0377 |

**Table 6. Stepwise Regression Analysis 2 - Cohort includes:**

| TBI Subjects >= 18 Years Old | | | | |
|---|---|---|---|---|
| Summary of Stepwise Selection - 39 Subjects | | | | |
| Variable | Parameter | Model | | |
| Step Entered | Estimate | R-Square | F Value | p-value |
| Intercept | 5.73685 | | | |
| 1 Serum_UCH_L1 | -0.30025 | 0.0821 | 8.82 | 0.0053 |
| 2 Serum_GFAP | 0.12083 | 0.1973 | 5.16 | 0.0291 |

**Table 7. Stepwise Regression Analysis 1 - Cohort includes:**

| TBI and A Subjects >= 18 Years Old | | | | |
|---|---|---|---|---|
| Summary of Stepwise Selection - 57 Subjects | | | | |
| Variable | Parameter | Model | | |
| Step Entered | Estimate | R-Square | F Value | p-value |
| Intercept | 8.04382 | | | |
| 1 Serum_UCH_L | -0.92556 | 0.1126 | 12.90 | 0.0007 |
| 2 Serum_MAP_2 | 1.07573 | 0.2061 | 5.79 | 0.0197 |
| 3 Serum_UCH-L1 | 0.01643 | 0.2663 | 4.35 | 0.0419 |
| +Serum_GFAP | | | | |

**Table 8. Stepwise Regression Analysis 2 - Cohort includes:**

| TBI Subjects >= 18 Years Old | | | | |
|---|---|---|---|---|
| Summary of Stepwise Selection - 44 Subjects | | | | |
| Variable | Parameter | Model | | |
| Step Entered | Estimate | R-Square | F Value | p-value |
| Intercept | 5.50479 | | | |
| 1 Serum UCH _L1 | -0.36311 | 0.0737 | 11.95 | 0.0013 |
| 2 SEX_Serum_GFAP | 0.05922 | 0.1840 | 5.09 | 0.0296 |
| 3 Serum_MAP_2 | 0.63072 | 0.2336 | 2.59 | 0.1157 |

### Example 3

The study of Example 2 is repeated with a moderate traumatic brain injury cohort characterized by GCS scores of between 9 and 11, as well as a mild traumatic brain injury cohort characterized by GCS scores of 12-15. Blood samples are obtained from each patient on arrival to the emergency department of a hospital within 2 hours of injury and measured by ELISA as described in Examples 1 and 2 for levels of GFAP in nanograms per milliliter. The results are compared to those of a control group who had not experienced any form of injury. Secondary outcomes included the presence of intracranial lesions in head CT scans.

Over 3 months 53 patients were enrolled: 35 with GCS 13-15, 4 with GCS 9-12 and 14 controls. The mean age was 37 years (range 18-69) and 66% were male. The mean GFAP serum level is 0 in control patients, 0.107 (0.012) in patients with GCS 13-15 and 0.366 (0.126) in GCS 9-12 (P<0.001). The difference between GCS 13-15 and controls is significant at P<0.001. In patients with intracranial lesions on CT, GFAP levels are 0.234 (0.055) compared to 0.085 (0.003) in patients without lesions (P<0.001). There is a significant increase in GFAP in serum following a MTBI compared to uninjured controls in both the mild and moderate groups. GFAP is also significantly associated with the presence of intracranial lesions on CT.

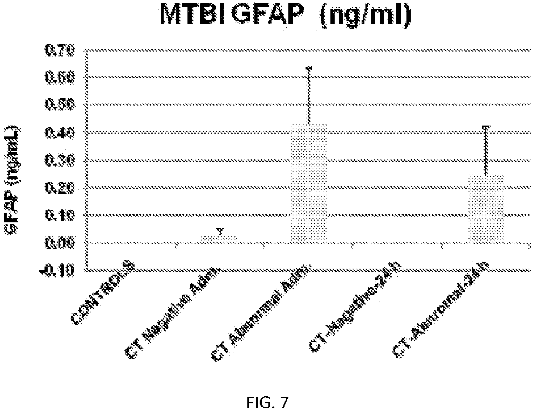

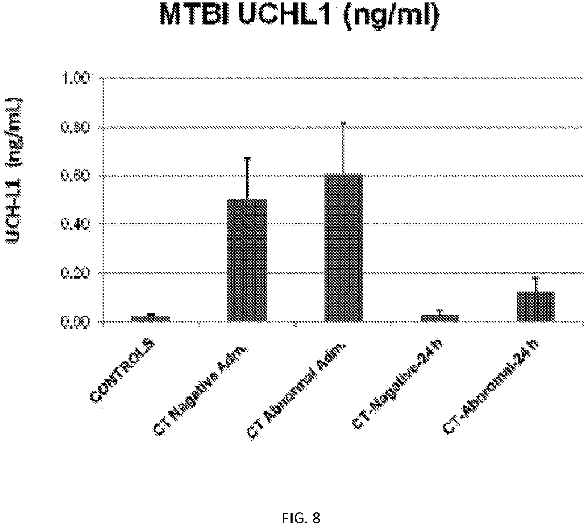

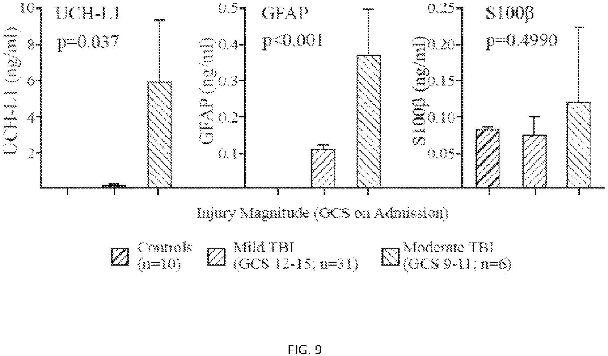

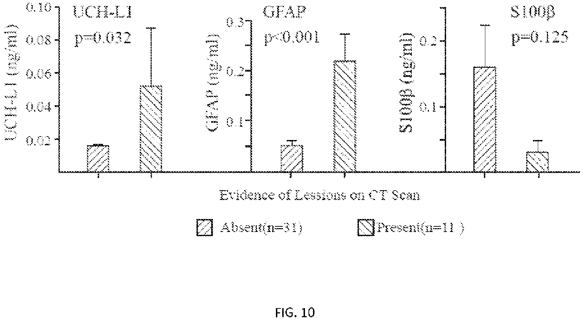

FIG. 7 shows GFAP concentration for controls as well as individuals in the mild/moderate traumatic brain injury cohort as a function of CT scan results upon admission and 24 hours thereafter. Simultaneous assays are performed in the course of this study for UCH-L1 biomarker. The UCH-L1 concentration derived from the same samples as those used to determine GFAP is provided FIG. 8. The concentration of UCH-L1 and GFAP as well as a biomarker not selected for diagnosis of neurological condition, S100β, is provided as a function of injury magnitude between control, mild, and moderate traumatic brain injury as shown in FIG. 9. FIG. 10 shows concentration of the same markers as depicted in FIG. 9 with respect to initial evidence upon hospital admission as a function of lesions observed in tomography scans. Through the simultaneous measurement of GFAP alone or UCH-L1 combined with GFAP values, rapid and quantifiable determination as to the severity of the brain injury is obtained consistent with GSC scoring and CT scanning yet in a more quantifiable, expeditious and economic process.

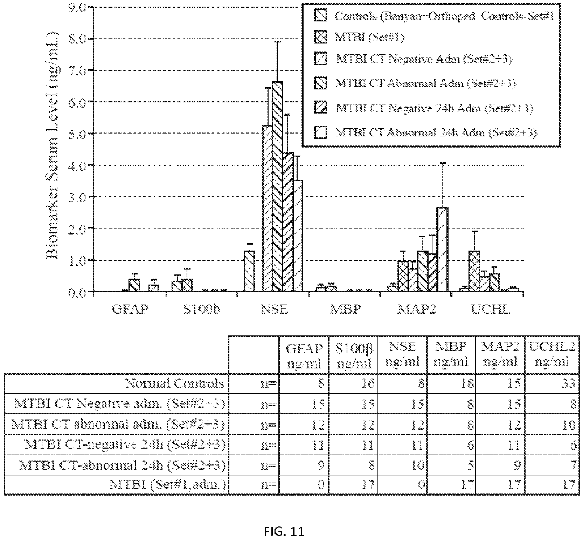

The samples of FIGs. 9 and 10 are also assayed for the levels of NES, MBP, and MAP2 also by ELISA essentially as described in Example 1. NSE and MAP2 are both elevated in MTBI serum as measured in samples obtained both at admission (within 2 hours of injury) and 24 hours later as depicted in FIG. 11.

Additionally, with a coupled assay for biomarkers indicative of neurological condition, the nature of the neurological abnormality is assessed and in this particular study suggestive of neuronal cell body damage. As with severe traumatic brain injury, gender variations are noted suggesting a role for hormonal anti-inflammatories as therapeutic candidates.

### Example 4

Controlled cortical impact *In vivo* model of TBI injury: A controlled cortical impact (CCI) device is used to model TBI on rats essentially as previously described (Pike et al, J Neurochem, 2001 Sep;78(6):1297-306, the contents of which are incorporated herein by reference). Adult male (280-300 g) Sprague-Dawley rats (Harlan: Indianapolis, IN) are anesthetized with 4% isoflurane in a carrier gas of 1:1 O₂/N₂O (4 min.) and maintained in 2.5% isoflurane in the same carrier gas. Core body temperature is monitored continuously by a rectal thermistor probe and maintained at 37±1°C by placing an adjustable temperature controlled heating pad beneath the rats. Animals are mounted in a stereotactic frame in a prone position and secured by ear and incisor bars. Following a midline cranial incision and reflection of the soft tissues, a unilateral (ipsilateral to site of impact) craniotomy (7 mm diameter) is performed adjacent to the central suture, midway between bregma and lambda. The dura mater is kept intact over the cortex. Brain trauma is produced by impacting the right (ipsilateral) cortex with a 5 mm diameter aluminum impactor tip (housed in a pneumatic cylinder) at a velocity of 3.5 m/s with a 1.6 mm compression and 150 ms dwell time. Sham-injured control animals are subjected to identical surgical procedures but do not receive the impact injury. Appropriate pre- and post-injury management is preformed to insure compliance with guidelines set forth by the University of Florida Institutional Animal Care and Use Committee and the National Institutes of Health guidelines detailed in the Guide for the Care and Use of Laboratory Animals. In addition, research is conducted in compliance with the Animal Welfare Act and other federal statutes and regulations relating to animals and experiments involving animals and adhered to principles stated in the "Guide for the Care and Use of Laboratory Animals, NRC Publication, 1996 edition."

At the appropriate time points (2, 6, 24 hours and 2, 3, 5 days) after injury, animals are anesthetized and immediately sacrificed by decapitation. Brains are quickly removed, rinsed with ice cold PBS and halved. The right hemisphere (cerebrocortex around the impact area and hippocampus) is rapidly dissected, rinsed in ice cold PBS, snap-frozen in liquid nitrogen, and stored at -80°C until used. For immunohistochemistry, brains are quick frozen in dry ice slurry, sectioned via cryostat (20 µm) onto SUPERFROST PLUS GOLD® (Fisher Scientific) slides, and then stored at -80°C until used. For the left hemisphere, the same tissue as the right side is collected. For western blot analysis, the brain samples are pulverized with a small mortar and pestle set over dry ice to a fine powder. The pulverized brain tissue powder is then lysed for 90 min at 4°C in a buffer of 50 mM Tris (pH 7.4), 5 mM EDTA, 1% (v/v) Triton X-100, 1 mM DTT, 1x protease inhibitor cocktail (Roche Biochemicals). The brain lysates are then centrifuged at 15,000xg for 5 min at 4°C to clear and remove insoluble debris, snap-frozen, and stored at -80°C until used.

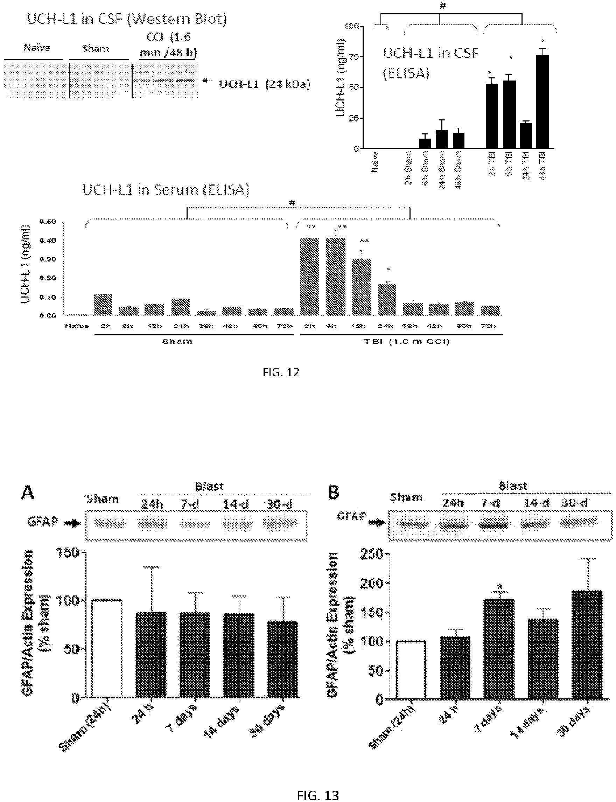

For gel electrophoresis and electroblotting, cleared CSF samples (7 µl) are prepared for sodium dodecyl sulfate-polyacrylamide gel electrophoresis (SDS-PAGE) with a 2X loading buffer containing 0.25 M Tris (pH 6.8), 0.2 M DTT, 8% SDS, 0.02% bromophenol blue, and 20% glycerol in distilled H₂O. Twenty micrograms (20 µg) of protein per lane are routinely resolved by SDS-PAGE on 10-20% Tris/glycine gels (Invitrogen, Cat #EC61352) at 130 V for 2 hours. Following electrophoresis, separated proteins are laterally transferred to polyvinylidene fluoride (PVDF) membranes in a transfer buffer containing 39 mM glycine, 48 mM Tris-HCl (pH 8.3), and 5% methanol at a constant voltage of 20 V for 2 hours at ambient temperature in a semi-dry transfer unit (Bio-Rad). After electro-transfer, the membranes are blocked for 1 hour at ambient temperature in 5% non-fat milk in TBS and 0.05% Tween-2 (TBST) then are incubated with the primary polyclonal UCH-L1 antibody in TBST with 5% non-fat milk at 1:2000 dilution as recommended by the manufacturer at 4°C overnight. This is followed by three washes with TBST, a 2 hour incubation at ambient temperature with a biotinylated linked secondary antibody (Amersham, Cat # RPN1177v1), and a 30 min incubation with Streptavidin-conjugated alkaline phosphatase (BCIP/NBT reagent: KPL, Cat # 50-81-08). Molecular weights of intact biomarker proteins are assessed using rainbow colored molecular weight standards (Amersham, Cat # RPN800V). Semi-quantitative evaluation of biomarker protein levels is performed via computer-assisted densitometric scanning (Epson XL3500 scanner) and image analysis with ImageJ software (NIH). UCH-L1 protein is readily detectable by western blot 48 hours after injury at levels above the amounts of UCH-L1 in sham treated and naive samples (FIG. 12).

ELISA is used to more rapidly and readily detect and quantitate UCH-L1 in biological samples in rats following CCI. For a UCH-L1 sandwich ELISA (swELISA), 96-well plates are coated with 100 µl/well capture antibody (500 ng/well purified rabbit anti-UCH-Ll, made in-house by conventional techniques) in 0.1 M sodium bicarbonate, pH 9.2. Plates are incubated overnight at 4°C, emptied and 300 µl/well blocking buffer (Startingblock T20-TBS) is added and incubated for 30 min at ambient temperature with gentle shaking. This is followed by either the addition of the antigen standard (recombinant UCH-L1) for standard curve (0.05 - 50 ng/well) or samples (3-10 µl CSF) in sample diluent (total volume 100 µl/well). The plate is incubated for 2 hours at room temperature, then washed with automatic plate washer (5 x 300 µl/well with wash buffer, TBST). Detection antibody mouse anti-UCH-Ll-HRP conjugated (made in-house, 50 µg/ml) in blocking buffer is then added to wells at 100µL/well and incubated for 1.5 h at room temperature, followed by washing. If amplification is needed, biotinyl-tyramide solution (Perkin Elmer Elast Amplification Kit) is added for 15 min at room temperature, washed then followed by 100 µl/well streptavidin-HRP (1:500) in PBS with 0.02% Tween-20 and 1% BSA for 30 min and then followed by washing. Lastly, the wells are developed with 100µl/well TMB substrate solution (Ultra-TMB ELISA, Pierce# 34028) with incubation times of 5-30 minutes. The signal is read at 652 nm with a 96-well spectrophotometer (Molecular Device Spectramax 190).

UCH-L1 levels of the TBI group (percussive injury) are significantly higher than the sham controls (p<0.01, ANOVA analysis) and the naive controls as measured by a swELISA demonstrating that UCH-L1 is elevated early in CSF (2h after injury) then declines at around 24 h after injury before rising again 48 h after injury (FIG. 12).

Similar results are obtained for UCH-L1 in serum. Blood (3-4 ml) is collected at the end of each experimental period directly from the heart using syringe equipped with 21 gage needle placed in a polypropylene tube and allowed to stand for 45 min to 1 hour at room temperature to form a clot. Tubes are centrifuged for 20 min at 3,000xg and the serum removed and analyzed by ELISA with results shown in FIG. 12. UCH-L1 levels of the TBI group are significantly higher than the sham group (p < 0.001, ANOVA analysis) and for each time point tested 2 h through 24 h respective to the same sham time points (p < 0.005, Student's T-test). UCH-L1 is significantly elevated after injury as early as 2h in serum.

### Example 5

Animal exposure to composite blast: Composite blast experiments are performed using the shock wave generator as described in Svetlov, SI, et al, J Trauma. 2010 Mar 2, doi: 10.1097/TA.0b013e3181bbd885, the contents of the entire manuscript of which are incorporated herein by reference.

Rats are anesthetized with 3-5% isoflurane in a carrier gas of oxygen using an induction chamber. At the loss of toe pinch reflex, the anesthetic flow is reduced to 1-3%. A nose cone continues to deliver the anesthetic gases. Isoflurane anesthetized rats are placed into a sterotaxic holder exposing only their head (body-armored setup) or in a holder allowing both head and body exposure. The head is allowed to move freely along the longitudinal axis and placed at the distance 5 cm from the exit nozzle of the shock tube, which is positioned perpendicular to the middle of the head (FIG. 2). The head is laid on a flexible mesh surface composed of a thin steel grating to minimize reflection of blast waves and formation of secondary waves that would potentially exacerbate the injury.

For pathomorphology and biomarker studies, animals are subjected to a single blast wave with a mean peak overpressure of 358 kPa at the head, and a total positive pressure phase duration of approximately 10 msec. This impact produces a non-lethal, yet strong effect.

For Analyses of biomarker levels in rat tissues, western blotting is performed on brain tissue samples homogenized on ice in western blot buffer as described previously in detail by Ringger NC, et al., J Neurotrauma, 2004;21:1443-1456, the contents of which are incorporated herein by reference. Samples are subjected to SDS-polyacrylamide gel electrophoresis and electroblotted onto PVDF membranes. Membranes are blocked in 10 mM Tris, pH 7.5, 100 mM NaCl, and 0.1% Tween-20 containing 5% nonfat dry milk for 60 min at room temperature. Anti-biomarker specific rabbit polyclonal and monoclonal antibodies are produced in the laboratory for use as primary antibodies. After overnight incubation with primary antibodies (1:2,000), proteins are detected using a goat anti-rabbit antibody conjugated to alkaline phosphatase (ALP) (1:10,000-15,000), followed by colorimetric detection system. Bands of interest are normalized by comparison to β-actin expression used as a loading control.

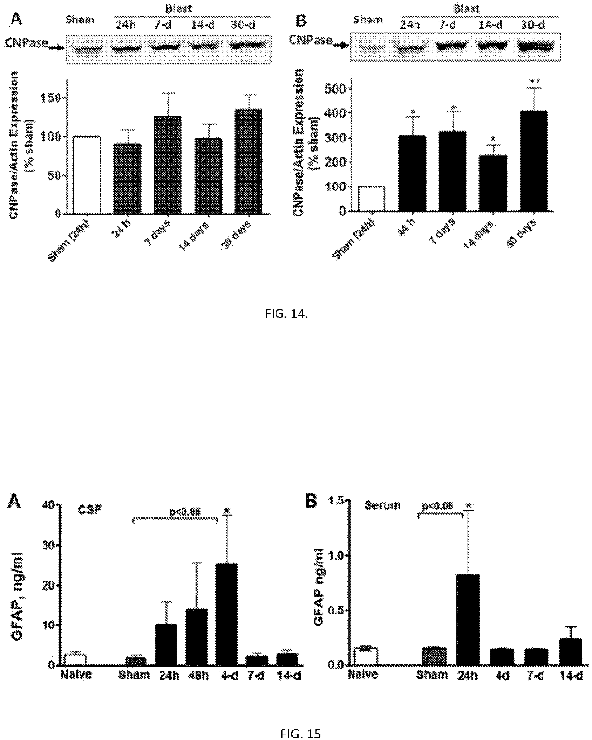

Severe blast exposure in the rat cortex demonstrates no significant increase of GFAP (FIG. 13A), in contrast to a significant GFAP accumulation in hippocampus (FIG. 13B). GFAP levels peak in hippocampus at 7 day after injury and persist up-to 30 day postblast (FIG. 13B). By contrast, CNPase accumulates significantly in the cortex between 7 and 30 days post-blast (FIG. 14A). The most prominent increase in CNPase expression is found in hippocampus demonstrating a nearly four-fold increase at 30 day after blast exposure (FIG. 14B).

Quantitative detection of GFAP and UCH-L1 in blood and CSF is determined by commercial sandwich ELISA. UCH-L1 levels are determined using a sandwich ELISA kit from Banyan Biomarkers, Inc., Alachua, FL. For quantification of glial fibrillary acid protein (GFAP), and neuron specific enolase (NSE) sandwich ELISA kits from BioVendor (Candler, NC) are used according to the manufacturer's instructions.

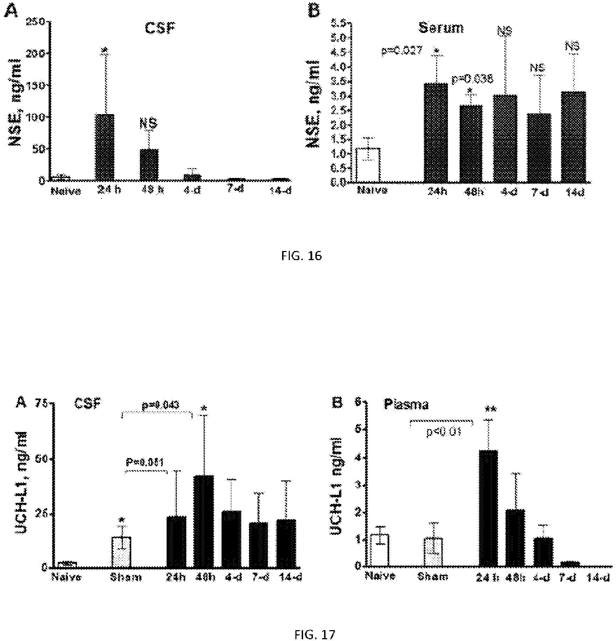

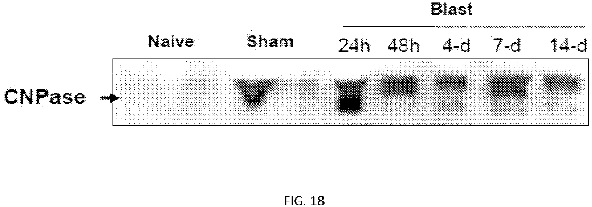

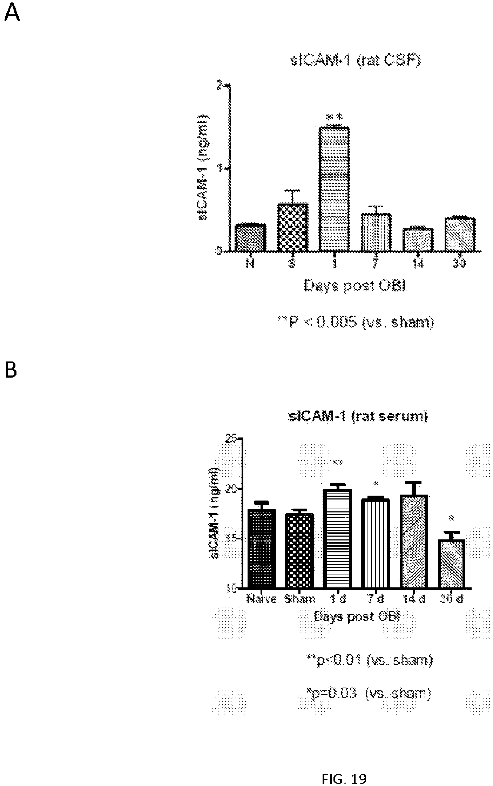

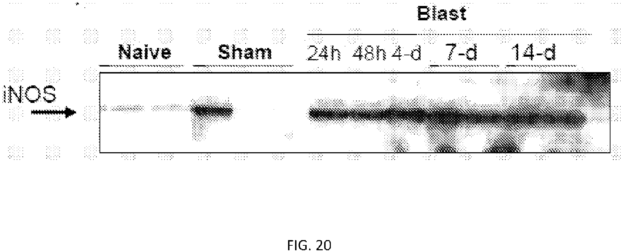

Increase of GFAP expression in brain (hippocampus) is accompanied by rapid and statistically significant accumulation in serum 24 h after injury followed by a decline thereafter (FIG. 15B). GFAP accumulation in CSF is delayed and occurs more gradually, in a time-dependent fashion (FIG. 15A). NSE concentrations are significantly higher at 24 and 48 hours post-blast period in exposed rats compared to naive control animals (FIG. 16). UCH-L1 levels trend to increased levels in CSF at 24 hours following injury (FIG. 17A). These levels increase to statistical significance by 48 hours. Plasma levels of UCH-L1 are increased to statistically significant levels by 24 hours followed by a slow decrease (FIG. 17B). Western blotting is used to detect levels of CNPase in rat CSF following blast injury. CNPase levels are increased at 24 hours after injury (FIG. 18). sICAM-1 levels are measured by ELISA following blast injury using the commercially available kit from R&D Systems, Inc. Minneapolis, MN essentially as per the manufacturer's instructions. Levels of sICAM-1 are increased to statically significant levels by one day post OBI in both CSF (FIG. 19A) and serum (FIG. 19B). iNOS levels are measured in rat plasma following blast overpressure injury. Levels of iNOS increase by day 4 with further increases observed by day 7 (FIG. 20).

### Example 6

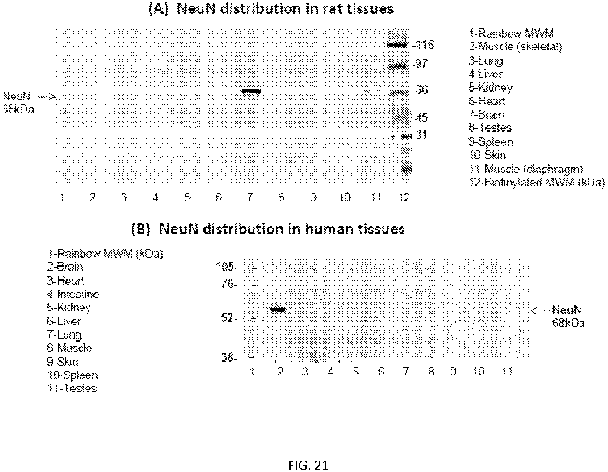

NeuN levels increase following traumatic brain injury. To examine the putative biomarker NeuN for tissue expression and levels in biological samples following inducement of traumatic brain injury as a model neurological condition, tissue samples are subjected to western blot analyses using biotin conjugated anti-NeuN antibody clone A60 from Millipore Corp., Billerica, MA with an avidin-HRP secondary antibody. The antibody shows cross reactivity to both human and rat NeuN. FIG. 21A illustrates that NeuN is primarily localized to the brain. Similarly, NeuN is found exclusive to the brain in humans (FIG. 21B).

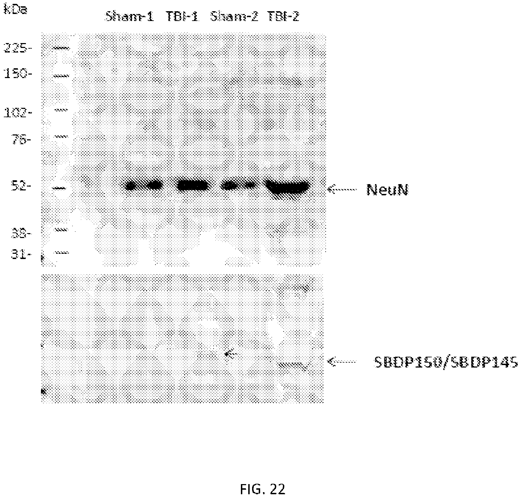

Rats are exposed to blast overpressure injury essentially as described in Example 5. NeuN levels are examined in CSF in either sham or TBI rats. The levels of NeuN are elevated following TBI as compared to sham treated animals (FIG. 22). This is similar in pattern to SBDPs 150 and 145 (FIG. 22).

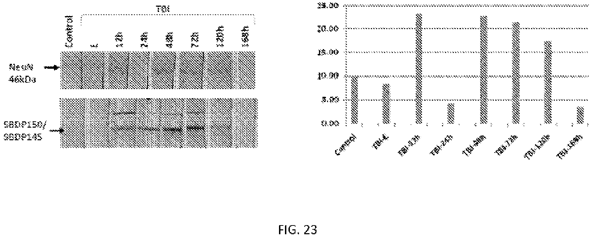

Humans suffering TBI as described in Example 2 are examined for NeuN levels in CSF. NeuN levels are increased at most time points as observed by western blot and quantified by densitometry as described herein (FIG. 23).

### Example 7

Levels of L-selectin, sICAM-1, β-NGF, Neuropilin-2, Resistin, Fractalkine, and Orexin are altered by experimental traumatic brain injury. Rats are subjected to primary blast OP exposure of controlled duration, peak pressure and transmitted impulse directed to various regions of the body essentially as described in Example 5, and samples of biomarkers are analyzed for biomarker levels by ELISA, antibody microarrays, and western blotting. The L-selectin antibody is L-Selectin (N-18) from Santa Cruz Biotechnology, Santa Cruz, CA. sICAM-1 is detected using a commercially available kit from R&D Systems, Inc. Minneapolis, MN essentially as per the manufacturer's instructions. β-NGF is detected using NGF (M-20) Antibody from Santa Cruz Biotechnology, Santa Cruz, CA. Neuropilin-2 is detected using neuropilin-2 (C-19) Antibody from Santa Cruz Biotechnology, Santa Cruz, CA. Resistin is detected using resistin (G-12) Antibody from Santa Cruz Biotechnology, Santa Cruz, CA. Fracktalkine is detected using fractalkine (B-1) Antibody from Santa Cruz Biotechnology, Santa Cruz, CA. The appropriate secondary antibodies are employed.

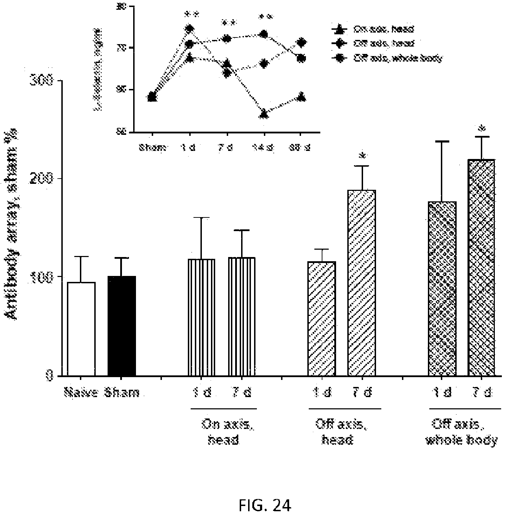

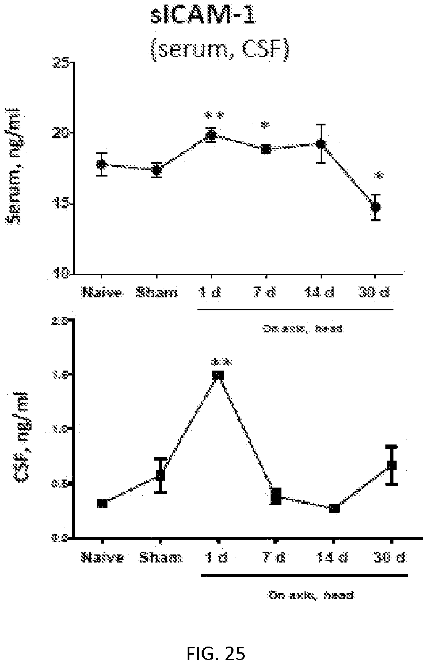

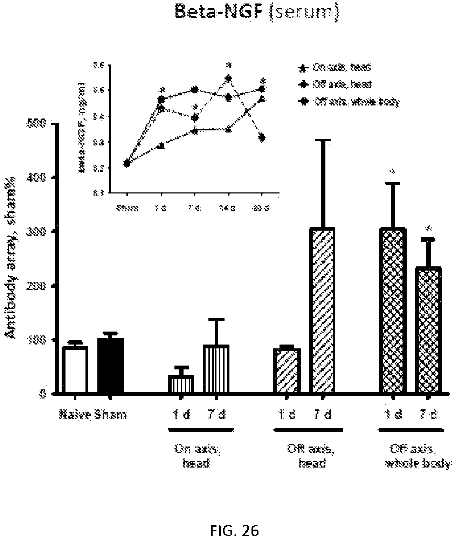

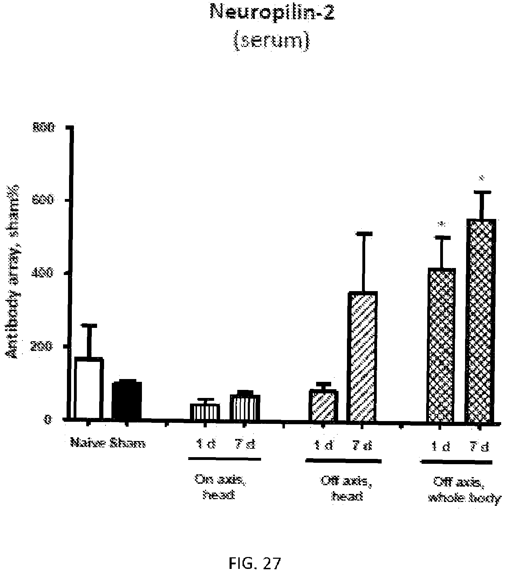

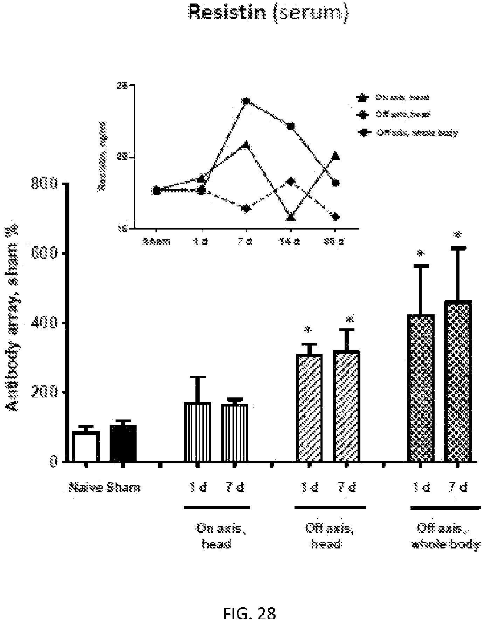

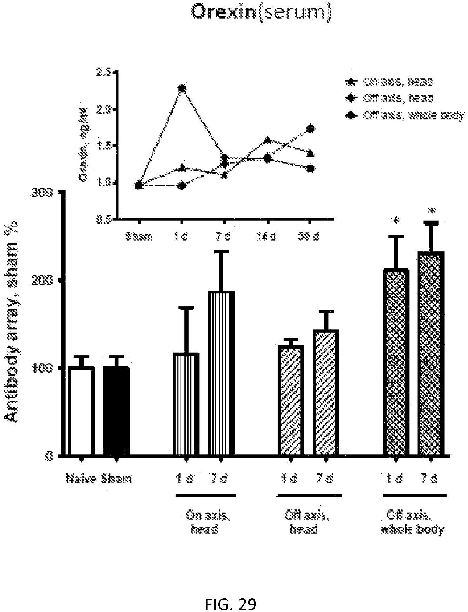

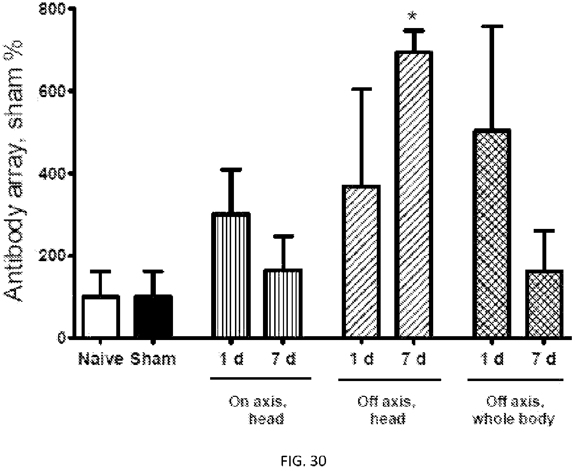

L-selectin (FIG. 24) and sICAM-1 (FIG. 25) accumulate substantially in rat blood 24 hours after blast and persist for 14 days post-blast. In CSF however, sICAM-1 content significantly increases at 24 h after injury, followed by a sharp decline (FIG. 25). β-NGF (FIG. 26) and Neuropilin-2 (FIG. 27) levels in serum are significantly elevated within the first week post-blast showing most pronounced changes when the total animal body is subjected to blast wave. Resistin significantly accumulates in rat serum 7 d after blast followed by a gradual decline (FIG. 28). Orexin content shows a drastic raise at 24 h after blast targeting total body, followed by gradual decline (FIG. 29). On the contrary, blast wave targeting only animal head causes a gradual raise of Orexin content through 30 d post exposure (FIG. 29). Fractalkine accumulates substantially in rat serum 24 h after blast and persists for 7 days post-blast, with remarkably high level following blast targeting total body (FIG. 30).

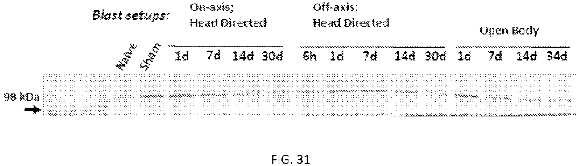

Levels of Neuropilin-2 are also measured in rat cerebellum by western blot. On axis head directed injury induces increased levels of Neuropilin-2 by one day after injury that progressively decreases over 30 days. Off axis injury produces a gradual increase in Neuropilin-2 peaking at 7 days and decreasing thereafter. Whole body blast produces similar Neuropilin-1 increases and decreases to that observed in on-axis injuries. (FIG. 31.)

### Example 8

*In vitro* drug candidate screening for neurotoxicity. Mouse, rat cortical or hippocampal primary neurons are cultured for 21 DIV, and the dose dependent responses of drugs are investigated. Cultured cells are exposed to various concentrations of: Glutamate (0.01 to 1000 µM) in 10 µM glycine both in HBSS; B) 0.01 to 100 µM Kainate in culture media; C) H₂O₂ (0.001 to 1000 µM) in culture media; C) Zinc (0.01 to 1000 µM) in culture media; D) U0126 (0.001 to 100 µM) in culture media; and E) and equal volume of culture media as a control. Glutamate treatment is performed for 30 minutes after which the cells are washed and the HBSS is replaced with culture media and analyzed. The remaining candidates are treated for 24 hours and analyzed. The levels of intracellular UCH-L1 and SBDP 145 are analyzed following cell lysis and screening of the lysates by ELISA using anti-UCH-Ll and SBDP 145 specific antibodies. The levels of UCH-L1 are increased following exposure particularly to Glutamate and H₂O₂.

### Example 9

Screening for neurotoxicity of developmental neurotoxicant compounds. ReNcell CX cells are obtained from Millipore (Temecula, CA). Cells frozen at passage 3 are thawed and expanded on laminin-coated T75 cm² tissue culture flasks (Corning, Inc., Corning, NY) in ReNcell NSC Maintenance Medium (Millipore) supplemented with epidermal growth factor (EGF) (20 ng/ml; Millipore) and basic fibroblast growth factor (FGF-2) (20 ng/ml; Millipore). Three to four days after plating (e.g., prior to reaching 80% confluency), cells are passaged by detaching with accutase (Millipore), centrifuging at 300 x g for 5 min and resuspending the cell pellet in fresh maintenance media containing EGF and FGF-2. For all experiments, cells are replated in laminin-coated costar 96-well plates (Corning, Inc., Corning, NY) at a density of 10,000 cells per well.

Immunocytochemical experiments are conducted to determine the level of UCH-L1 and SBDP 145 in cells prior to and following 24 hours of exposure to 1nM-100µM of methyl mercury chloride, *trans*-retinoic acid, D-amphetamine sulfate, cadmium chloride, dexamethasone, lead acetate, 5,5-diphenylhydantoin, and valproic acid essentially as described in Breier JM et al, Toxicological Sciences, 2008; 105(1):119-133, the contents of which are incorporated herein by reference. Cells are fixed with a 4% paraformaldehyde solution and permeabilized in blocking solution (5% normal goat serum, 0.3% Triton X-100 in phosphate-buffered saline). Fluorescein labeled anti- UCH-L1 Antibody #3524 (Cell Signaling Technology, Danvers, MA) is incubated with the fixed cells overnight at 4°C overnight and visualized using a Nikon TE200 inverted fluorescence microscope with a 20x objective. Images are captured using an RT Slider camera (Model 2.3.1., Diagnostic Instruments, Inc., Sterling Heights, MI) and SPOT Advantage software (Version 4.0.9, Diagnostic Instruments, Inc.).

### Examples 10-14

Acute oral *In vivo* drug candidate screening for neurotoxicity. Female Sprague-Dawley rats (Charles River Laboratories, Inc., Wilmington, MA) are dosed with methamphetamine (40 mg/kg as four 10mg/kg intraperitoneal injections (i.p.) (n=8), kainic acid (1.2 nM solution injected i.p.), MPTP (30 mg/kg, s.c.), dizocilpine (0.1 mg/kg, i.p.) or the chemotherapeutic cisplatin (10 mg/kg (single i.p. injection)) (n=4). Anesthesia is performed with intraperitoneal injections of pentobarbital (50 mg/kg). The test substance can also be administered in a single dose by gavage using a stomach tube or a suitable intubation cannula. Animals are fasted prior to dosing. A total of four to eight animals of are used for each dose level investigated.

At 30, 60, 90, and 120 minutes following dosing, the rats are sacrificed by decapitation and blood is obtained by cardiac puncture. The levels of bio fluid UCH-L1 and SBDP 150 and GFAP are analyzed by sandwich ELISA or western blot by using UCH-L1 and SBDP 150 and GFAP specific antibodies. Relative to control animals, neurotoxic levels of methamphetamine induce increase CSF concentrations of both UCH-L1 and SBDP 150 and GFAP. Cisplatin, kainic acid, MPTP, and dizocilpine increase UCH-L1, GFAP, and SBDP150 levels.

### Example 15

Middle cerebral artery occlusion (MCAO) injury model: Rats are incubated under isoflurane anesthesia (5% isoflurane *via* induction chamber followed by 2% isoflurane *via* nose cone), the right common carotid artery (CCA) of the rat is exposed at the external and internal carotid artery (ECA and ICA) bifurcation level with a midline neck incision. The ICA is followed rostrally to the pterygopalatine branch and the ECA is ligated and cut at its lingual and maxillary branches. A 3-0 nylon suture is then introduced into the ICA *via* an incision on the ECA stump (the suture's path was visually monitored through the vessel wall) and advanced through the carotid canal approximately 20 mm from the carotid bifurcation until it becomes lodged in the narrowing of the anterior cerebral artery blocking the origin of the middle cerebral artery. The skin incision is then closed and the endovascular suture left in place for 30 minutes or 2 hours. Afterwards the rat is briefly reanesthetized and the suture filament is retracted to allow reperfusion. For sham MCAO surgeries, the same procedure is followed, but the filament is advanced only 10 mm beyond the internal-external carotid bifurcation and is left in place until the rat is sacrificed. During all surgical procedures, animals are maintained at 37 ± 1°C by a homeothermic heating blanket (Harvard Apparatus, Holliston, MA, U.S.A.). At the conclusion of each experiment, if the rat brains show pathologic evidence of subarachnoid hemorrhage upon necropsy they are excluded from the study. Appropriate pre- and post-injury management is preformed to insure compliance with all animal care and use guidelines.

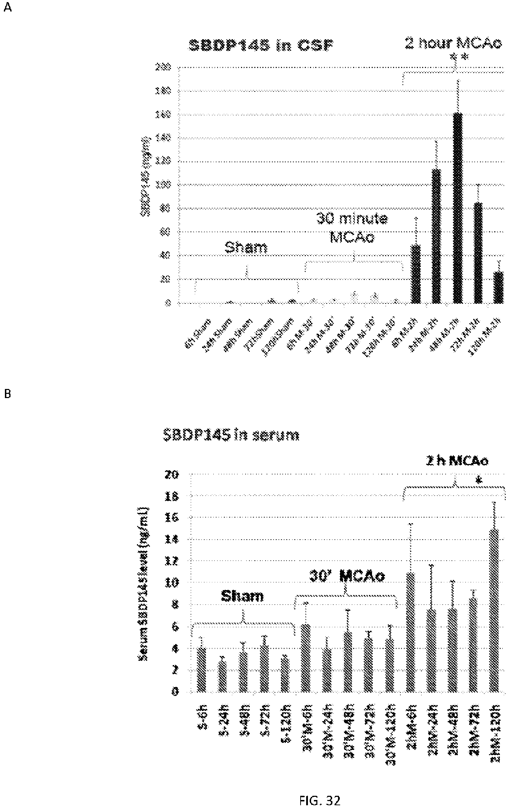

Spectrin breakdown products are analyzed following rat MCAO challenge by procedures similar to those described in U.S. Patent No. 7,291,710, the contents of which are incorporated herein by reference. FIG. 32 demonstrates that levels of SBDP145 in both serum and CSF are significantly (p<0.05) increased at all time points studied following severe (2hr) MCAO challenge relative to mild (30 min) challenge. Similarly, SBDP120 demonstrates significant elevations following severe MCAO challenge between 24 and 72 hours after injury in CSF (FIG. 7). However, levels of SBDP120 in serum are increased following severe challenge relative to mild challenge at all time points between 2 and 120 hours. In both CSF and Serum both mild and severe MCAO challenge produces increased SPBP120 and 140 relative to sham treated subjects.

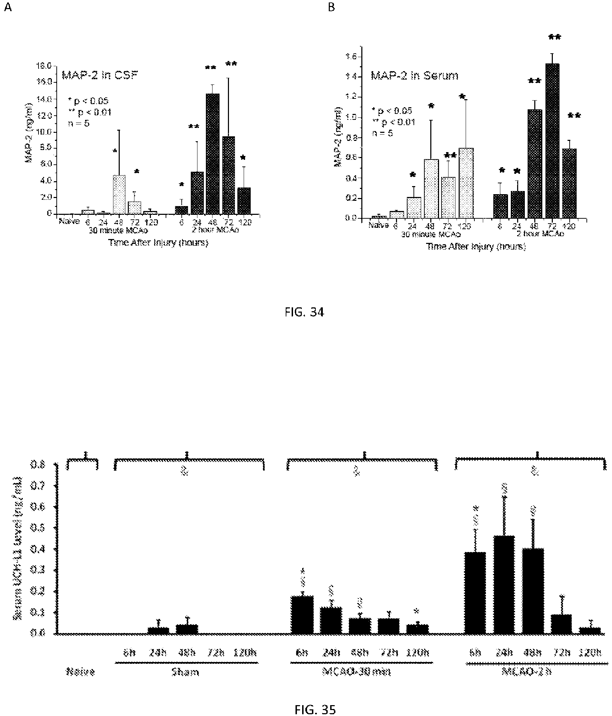

Microtubule Associated Protein 2 (MAP2) is assayed as a biomarker in both CSF and serum following mild (30 min) and severe (2 hr) MCAO challenge in subjects by ELISA or western blotting essentially as described herein. Antibodies to MAP2 (MAP-2 (E-12)) are obtained from Santa Cruz Biotechnology, Santa Cruz, CA. These antibodies are suitable for both ELISA and western blotting procedures and are crossreactive to murine and human MAP2. Levels of MAP2 are significantly (p<0.05) increased in subjects following mild MCAO challenge relative to naive animals in both CSF and serum (FIG. 34). Similar to UCH-L1 and SBDPs, severe challenge (2 hr) produces much higher levels of MAP2 in both samples than mild challenge (30 min).

ELISA is used to rapidly and readily detect and quantitate UCH-L1 in biological samples. For a UCH-L1 sandwich ELISA (swELISA), 96-well plates are coated with 100 µl/well capture antibody (500 ng/well purified rabbit anti-UCH-Ll, made in-house by conventional techniques) in 0.1 M sodium bicarbonate, pH 9.2. Plates are incubated overnight at 4°C, emptied and 300 µl/well blocking buffer (Startingblock T20-TBS) is added and incubated for 30 min at ambient temperature with gentle shaking. This is followed by either the addition of the antigen standard (recombinant UCH-L1) for standard curve (0.05 - 50 ng/well) or samples (3-10 µl CSF) in sample diluent (total volume 100 µl/well). The plate is incubated for 2 hours at room temperature, then washed with automatic plate washer (5 x 300 µl/well with wash buffer, TBST). Detection antibody mouse anti-UCH-Ll-HRP conjugated (made in-house, 50 µg/ml) in blocking buffer is then added to wells at 100µL/well and incubated for 1.5 h at room temperature, followed by washing. If amplification is needed, biotinyl-tyramide solution (Perkin Elmer Elast Amplification Kit) is added for 15 min at room temperature, washed then followed by 100 µl/well streptavidin-HRP (1:500) in PBS with 0.02% Tween-20 and 1% BSA for 30 min and then followed by washing. Lastly, the wells are developed with 100µl/well TMB substrate solution (Ultra-TMB ELISA, Pierce# 34028) with incubation times of 5-30 minutes. The signal is read at 652 nm with a 96-well spectrophotometer (Molecular Device Spectramax 190).

Following MCAO challenge the magnitude of UCH-L1 in serum is dramatically increased with severe (2h) challenge relative to a more mild challenge (30 min). (FIG. 35) The more severe 2 h MCAO group UCH-L1 protein levels are 2-5 fold higher than 30 min MCAO (p < 0.01, ANOVA analysis). Group comparison of UCH-L1 levels by ANOVA indicates that all groups at all time points combined (naive, sham, 30 min MCAO and 2 h MCAO) are significantly different from each other (§ p<0.001). There are also statistically significant differences for 6 h, 24 h, and 48 h time points overall between all groups (& p<0.001). For time points 6 h and 120 h for MCAO-30 min and 6 h for MCAO-2 h, UCH-L1 levels are significantly different from their respective sham time groups (*p<0.05).

### Example 16

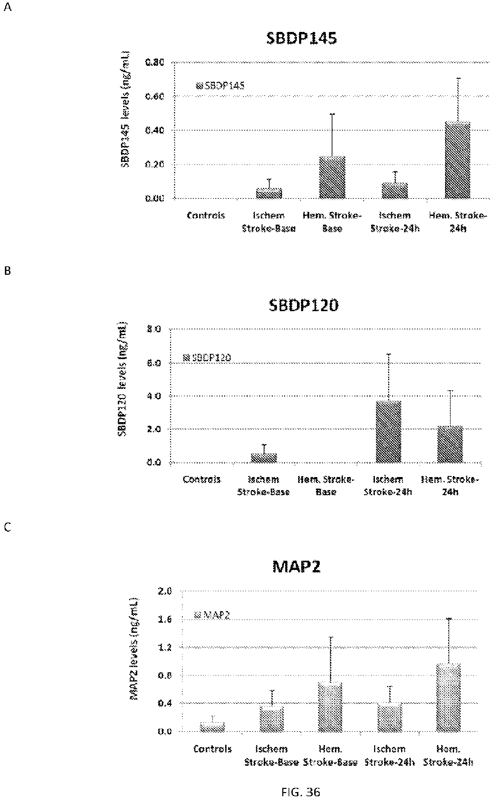

Biomarker levels correlate with stroke injury in human subjects. Samples are commercially obtained from HeartDrug, Inc., Towson, MD. Plasma samples in citrate as the anticoagulant are taken from human patients suffering ischemic (n =15) or hemorrhagic (n = 9) stroke as well as citrate plasma controls (no known stroke symptoms, n = 10) at patient admission (baseline) and approximately 24 hrs after symptom onset. Assays of SBDP145, SBDP120 and MAP2 levels are performed by ELISA essentially as described in Example 16. As shown in FIG. 36, SBDP 145, SBDP 120 and MAP-2 are elevated following stroke with the most notable trends occurring in hemorrhagic stroke patients.

### Example 17

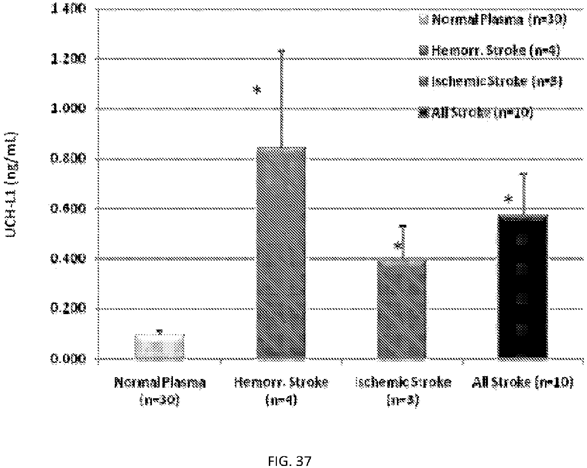

Biomarker levels in biological samples obtained from human stroke patients. Samples of citrated plasma are obtained from blood draws performed within 24 hrs of onset of stroke symptoms of patients (n=10: 5 ischemic stroke, 5 hemorrhagic stroke). UCH-L1 as measured by ELISA as described herein is significantly elevated in blood from stroke patients as compared to normal controls for both hemorrhagic and ischemic groups (FIG. 37). Differences between ischemic and control patients demonstrate a trend P=0.2 but did not reach statistical significance with this small sample set. A preliminary ROC analysis yields a UC of 0.98 (p > .003). UCH-L1 discriminates between hemorrhagic and ischemic stroke.

Methods involving conventional biological techniques are described herein. Such techniques are generally known in the art and are described in detail in methodology treatises such as Molecular Cloning: A Laboratory Manual, 2nd ed., vol. 1-3, ed. Sambrook et al., Cold Spring Harbor Laboratory Press, Cold Spring Harbor, N.Y., 1989; and Current Protocols in Molecular Biology, ed. Ausubel et al., Greene Publishing and Wiley-Interscience, New York, 1992 (with periodic updates). Immunological methods (e.g., preparation of antigen-specific antibodies, immunoprecipitation, and immunoblotting) are described, e.g., in Current Protocols in Immunology, ed. Coligan et al., John Wiley & Sons, New York, 1991; and Methods of Immunological Analysis, ed. Masseyeff et al., John Wiley & Sons, New York, 1992. The entire contents of each of the aforementioned publications are incorporated herein by reference as if each were explicitly included herein in their entirety.

Patent documents and publications mentioned in the specification are indicative of the levels of those skilled in the art to which the invention pertains. These documents and publications are incorporated herein by reference to the same extent as if each individual document or publication was specifically and individually incorporated herein by reference.

The foregoing description is illustrative of particular embodiments of the invention, but is not meant to be a limitation upon the practice thereof. The following claims, including all equivalents thereof, are intended to define the scope of the invention.

## Claims

1. A process for determining the neurological condition of a subject, comprising:
measuring a quantity of a biomarker in a sample obtained from the subject wherein the quantity of the biomarker correlates with the presence or absence of a neurological condition, wherein the biomarker is L-selectin, ICAM, β-NGF, neuropilins or resistin.

2. The process of claim 1, wherein the neurological condition is traumatic brain injury.

3. The process of claim 2, wherein the neurological condition is caused by a blast.

4. The process of claim 2 or 3, wherein the sample is serum.

5. The process of any one of claims 2-4, wherein the biomarker is L-selectin or ICAM.

6. The process of any one of claims 2-4, wherein the biomarker is β-NGF or neuropilins.

7. The process of any one of claims 2-4, wherein the biomarker is resistin.

8. The process of any one of claims 2-7, wherein the biomarker is detected by ELISA, antibody microarrays, or western blotting.

9. The process of any one of claims 2-8, wherein the biomarker is detected by ELISA.

10. The process of any one of claims 2-9, wherein the biomarker is detected using an antibody.

11. The process of claim 1, wherein the neurological condition is traumatic brain injury caused by a blast, wherein the sample is serum, wherein the biomarker is L-selectin, sICAM-1, β-NGF, neuropilin-2 or resistin, wherein the biomarker is detected by ELISA, antibody microrrays or western blotting and wherein
the quantity of L-selectin and sICAM-1 is measured between 24 hours after blast and 14 days after blast,
the quantity of β-NGF and neuropilin-2 is measured within the first week after blast,
the quantity of resistin is measured 7 days after blast.
